# FASCICULE DE BREVET EUROPEEN

(11) **EP 4 051 349 B1**
(45) Date de publication et mention de la délivrance du brevet: **09.08.2023**
(21) Numéro de dépôt: 20797482.5
(22) Date de dépôt: 30.10.2020
(51) Int. Cl.: A61M 11/00, B05B 11/00, A61M 15/08

(54) **DISPOSITIF D'ASSISTANCE À L'UTILISATION D'UN DISPOSITIF DE DISTRIBUTION DE PRODUIT**
VORRICHTUNG ZUR UNTERSTÜTZUNG DER VERWENDUNG EINER VORRICHTUNG ZUR ABGABE EINES PRODUKTS
DEVICE FOR ASSISTING WITH THE USE OF A DEVICE FOR DISPENSING A PRODUCT

(30) Priorité: 31.10.2019 FR 1912304
(43) Date de publication de la demande: 07.09.2022
(73) Titulaire: NEMERA LA VERPILLIERE, 38290 La Verpillière (FR)
(72) Inventeur: DECOCK, Thierry, 69007 Lyon (FR); PINTO, Joris, 69800 Saint-Priest (FR); GUIMARD, Lenaic, 38500 Saint-Cassien (FR); PAINCHAUD, Gaëtan, 69340 Francheville (FR); VERNANT, Olivier, 38280 Anthon (FR)
(74) Mandataire: LLR
(86) Numéro de dépôt international: PCT/EP2020/080557
(87) Numéro de publication internationale: WO 2021/084092

(56) Documents cités:
- EP-A1- 2 436 415
- US-A1- 2010 084 433
- US-A1- 2018 060 527

## Description

L'invention concerne un dispositif d'assistance à l'utilisation d'un dispositif de distribution de produit.

On connaît déjà dans l'état la technique, notamment d'après le document US-A1-2010/084433 un dispositif d'assistance à l'utilisation d'un dispositif de distribution de produit comprenant des moyens de verrouillage qui empêchent la distribution de produit par le dispositif de distribution en bloquant le déplacement d'une partie mobile d'une pompe du dispositif de distribution afin d'empêcher une personne non autorisée d'utiliser le dispositif de distribution ou d'empêcher la délivrance de produit pendant un laps de temps donné. Le dispositif d'assistance comprend des moyens de blocage des moyens de verrouillage en position de déverrouillage. Lorsque les moyens de blocage maintiennent les moyens de verrouillage en position de déverrouillage, l'unique moyen de désengager les moyens de blocage, et donc de repasser en position de verrouillage, consiste à actionner le dispositif de distribution de produit et donc à délivrer une dose de produit. Ainsi une activation du dispositif de distribution est nécessaire afin de permettre aux moyens de verrouillage de revenir en position de verrouillage.

Un tel dispositif présente des inconvénients. Notamment, une dose de produit est gaspillée lorsqu'un reverrouillage du dispositif de distribution est souhaité alors que la délivrance d'une dose de produit n'est, elle, pas voulue. En outre, il n'est pas possible de prévoir des reverrouillages automatiques indépendants de la distribution de produit.

L'invention a notamment pour but de fournir un dispositif d'assistance à l'utilisation d'un dispositif de distribution de produit permettant de surmonter au moins une partie de ces inconvénients.

À cet effet, l'invention a notamment pour objet un dispositif d'assistance à l'utilisation d'un dispositif de distribution d'un produit, la distribution du produit étant mise en oeuvre par déplacement ou déformation d'une partie mobile du dispositif de distribution entre une position de repos et une position d'activation, le dispositif d'assistance comprenant:
- des moyens de solidarisation au dispositif de distribution de produit,
- des moyens de verrouillage aptes à empêcher la distribution du produit,
- un actionneur configuré pour faire passer les moyens de verrouillage d'une position de verrouillage dans laquelle ils empêchent la distribution du produit à une position de déverrouillage dans laquelle ils n'empêchent pas la distribution du produit,
- des moyens de retour configurés pour faire passer les moyens de verrouillage de la position de déverrouillage à la position de verrouillage, en dehors de la position d'activation du dispositif de distribution du produit,
- des moyens de blocage configurés pour bloquer les moyens de verrouillage en position de déverrouillage,
- des premiers moyens de désengagement des moyens de blocage configurés pour être déclenchés lors de l'activation du dispositif de distribution de produit, et
- des seconds moyens de désengagement des moyens de blocage configurés pour être déclenchés indépendamment de l'activation du dispositif de distribution de produit.

Il est ainsi possible de reverrouiller le système sans avoir à délivrer une dose de produit, ce qui permet d'éviter un gaspillage de produit par exemple dans le cas où on aurait déverrouillé le système mais qu'on ne souhaiterait finalement pas délivrer une dose. C'est par exemple le cas lors d'un déverrouillage accidentel. Cela est également avantageux en ce qu'il est possible de prévoir que les moyens de verrouillage repassent en position de verrouillage en fonction d'un paramètre prédéterminé indépendamment de l'activation du dispositif de distribution, par exemple en fonction d'un temps de latence trop important depuis un déverrouillage ou une distribution d'une dose de produit ou en raison d'une mauvaise inclinaison du dispositif de distribution.

Dans un mode de réalisation de l'invention, les moyens de verrouillage peuvent être directement intégrés au dispositif de distribution, par exemple en interaction avec une pompe compris dans ce même dispositif de distribution. Le dispositif d'assistance serait alors semblable à un dispositif réutilisable pouvant s'adapter à différents dispositifs de distribution successifs comprenant différents types de moyens de verrouillage. Le dispositif d'assistance serait alors durable et d'une praticité importante.

De préférence, l'actionneur comprend au moins un fil rétractable.

L'utilisation d'un tel fil rétractable présente plusieurs avantages. Un fil rétractable ne nécessite que très peu d'énergie d'activation pour faire passer les moyens de verrouillage de la position de verrouillage à la position de déverrouillage. En outre, un tel fil rétractable est intéressant puisqu'il présente une faible usure et ne devra donc pas être remplacé trop souvent. De plus, il présente une grande fiabilité d'activation via le contrôle du courant électrique qui lui est appliqué.

Selon un autre mode de réalisation, l'actionneur comprend un moteur.

L'utilisation d'un moteur en tant qu'actionneur permet des déplacements plus importants des moyens de verrouillage (par exemple par l'intégration d'une roue ou d'une crémaillère pilotée par ce moteur) par rapport à un autre moyen tel qu'un fil rétractable. De même, si le moteur est rotatif il peut être possible de prévoir qu'un sens de rotation du moteur permet un déplacement des moyens de verrouillage vers la position de déverrouillage alors que le sens contraire de rotation du moteur renvoie les moyens de verrouillage vers la position de verrouillage. En outre, on peut prévoir la présence de moyens de contrôle aptes à commander et/ou réaliser un suivi des actions du moteur.

Avantageusement, les seconds moyens de désengagement comprennent au moins un fil rétractable.

Comme indiqué précédemment, l'utilisation d'un tel fil rétractable présente plusieurs avantages. Un fil rétractable permet d'utiliser une faible énergie d'activation pour faire passer les moyens de verrouillage de la position de verrouillage à la position de déverrouillage. En outre, un tel fil rétractable est intéressant puisqu'il présente une faible usure et ne devra donc pas être remplacé trop souvent. De plus, il présente une grande fiabilité d'activation via le contrôle du courant électrique qui lui est appliqué.

Avantageusement, les seconds moyens de désengagement comprennent un moteur.

L'utilisation d'un moteur en tant que seconds moyens de désengagement permet des déplacements plus importants des moyens de verrouillage (par exemple par l'intégration d'une roue ou d'une crémaillère pilotée par ce moteur) par rapport à un autre moyen tel qu'un fil rétractable. En outre, on peut prévoir la présence de moyens de contrôle aptes à commander et/ou réaliser un suivi des actions du moteur.

De préférence, les seconds moyens de désengagement sont configurés pour être activés après qu'un temps prédéterminé se soit écoulé à partir d'un passage des moyens de verrouillage en position de déverrouillage.

Cela permet, par exemple, de faire repasser automatiquement les moyens de verrouillage en position de verrouillage après qu'un déverrouillage ait eu lieu. Ceci est notamment avantageux lorsque le passage en position de déverrouillage des moyens de verrouillage a été fait de manière accidentelle. On réduit de cette façon le risque qu'un dispositif de distribution qui a été déverrouillé et laissé sans surveillance puisse être utilisé par une personne à laquelle le produit n'est pas destiné et pour laquelle le produit pourrait représenter un danger, par exemple un enfant. On réduit également le risque qu'une distribution de produit ait lieu de manière accidentelle, par exemple dans un sac ou dans une poche. De cette façon, on évite de gaspiller du produit et on évite que le patient puisse ne pas avoir accès à son produit si le dispositif est conçu pour rester en position de verrouillage pendant un certain laps de temps après la distribution d'une dose de produit. On peut par exemple prévoir que le dispositif d'assistance comprend des moyens de contrôle aptes à vérifier les conditions permettant l'activation des seconds moyens de désengagement. Par exemple, ces moyens de contrôle pourraient comprendre un détecteur d'une position de déverrouillage et/ou un compteur de temps apte à mesurer une durée de temps écoulée après un déverrouillage du dispositif de distribution.

Avantageusement, le dispositif d'assistance comprend en outre au moins un capteur d'inclinaison du dispositif de distribution, les seconds moyens de désengagement étant configurés pour être activés lorsque, les moyens de verrouillage étant en position de déverrouillage, une mesure d'inclinaison du dispositif de distribution mesurée par le capteur d'inclinaison atteint un seuil prédéterminé pendant un laps de temps prédéterminé.

Ainsi, il est possible de faire repasser les moyens de verrouillage en position de verrouillage si la mesure d'inclinaison est égale à un seuil prédéterminé qui traduit une inclinaison du dispositif de distribution incompatible avec une bonne distribution du produit. De manière générale, l'inclinaison du dispositif de distribution peut être mesurée en déterminant le décalage angulaire entre un axe longitudinal principal du dispositif de distribution et un axe de gravité.

On peut aussi prévoir que l'inclinaison du dispositif de distribution soit mesurée de manière indirecte, par exemple en mesurant une inclinaison du dispositif d'assistance et en en déduisant l'inclinaison du dispositif de distribution puisque le décalage angulaire entre un axe longitudinal principal du dispositif d'assistance et un axe longitudinal principal du dispositif de distribution est constant et connu pour un dispositif d'assistance associé à un dispositif de distribution donné. De préférence, les axes longitudinaux principaux des dispositifs de distribution et d'assistance sont parallèles, voire confondus. Le seuil d'inclinaison pourra être défini par rapport à un plan ou axe quelconque, par exemple par rapport à un axe vertical ou un axe horizontal.

Selon un mode de réalisation avantageux, les moyens de verrouillage sont configurés pour passer de la position de déverrouillage à la position de verrouillage en suivant un mouvement de rotation.

Ainsi, dans le cas par exemple où les seconds moyens de désengagement comprennent un fil rétractable, il est plus aisé de prévoir un fil rétractable plus long, permettant d'obtenir, pour un courant électrique donné, une course plus longue des moyens de verrouillage au cours de leur rotation et donc un reverrouillage plus sûr avec peu d'énergie utilisée. On peut prévoir que la rotation des moyens de verrouillage est une rotation autour d'un axe longitudinal principal du dispositif d'assistance.

On peut également prévoir que les moyens de verrouillage sont configurés pour passer de la position de verrouillage à la position de déverrouillage en suivant un mouvement de rotation. Alternativement, il est possible de prévoir que les moyens de verrouillage sont configurés pour que le passage de la position de verrouillage à la position de déverrouillage et/ou le passage de la position de déverrouillage à la position de verrouillage se fasse par un autre mouvement qu'un mouvement de rotation, par exemple par un mouvement de translation ou par un mouvement comprenant au moins une composante de rotation et/ou au moins une composante de translation.

Avantageusement, le dispositif d'assistance comprend au moins un cran apte à empêcher le retour de la partie mobile du dispositif de distribution vers la position de repos après un déplacement selon une course prédéterminée de la partie mobile du dispositif de distribution, la course prédéterminée étant plus courte qu'une course totale correspondant à la position d'activation du dispositif de distribution.

Ainsi, on réduit le risque qu'une activation partielle du dispositif de distribution soit réalisée et donc qu'une partie seulement de la dose de produit devant être distribuée soit effectivement distribuée. Une telle distribution partielle présente le risque qu'un utilisateur, par exemple un patient, reçoive une dose insuffisante de produit. On peut prévoir que le cran a une forme complémentaire d'un cran présent sur la partie mobile du dispositif de distribution, ces crans étant agencés pour venir en prise lorsque la partie mobile du dispositif de distribution a parcouru la course prédéterminée. On peut prévoir que le dispositif d'assistance comprend des moyens de comptage d'un nombre de distribution de dose de produit, ces moyens de comptage étant agencés pour considérer qu'une distribution de produit a eu lieu uniquement après que la partie mobile du dispositif de distribution ait parcouru une course plus grande que la course prédéterminée, et de préférence ait parcouru la course totale.

### Brève description des figures

Nous allons maintenant présenter plusieurs modes de réalisation de l'invention donnés à titre d'exemples non limitatifs à l'appui des figures annexées sur lesquelles :
[Fig. 1] la figure 1 est une vue en perspective d'un dispositif d'assistance et d'un dispositif de distribution selon un premier mode de réalisation de l'invention.
[Fig. 2] les figures 2A et 2B sont des vues respectivement en perspective et en plan d'une partie du dispositif d'assistance du premier mode de réalisation, représentant notamment des moyens de verrouillage et des moyens de blocage des moyens de verrouillage.
[Fig. 3] les figures 3A à 3C sont des vues en perspective d'une partie du dispositif d'assistance du premier mode de réalisation, dans lesquelles les moyens de verrouillage sont respectivement en position de verrouillage, bloqués en position de déverrouillage par des moyens de blocage, et en cours de retour vers la position de verrouillage.
[Fig. 4] les figures 4A à 4H sont des vues en coupe d'un dispositif d'assistance selon un second mode de réalisation de l'invention à différents états de verrouillage et déverrouillage des moyens de verrouillage.
[Fig. 5] les figures 5A à 5H sont des vues en coupe d'un dispositif d'assistance selon un troisième mode de réalisation de l'invention à différents états de verrouillage et déverrouillage des moyens de verrouillage.
[Fig. 6] la figure 6 est une vue éclatée en perspective d'une partie d'un dispositif d'assistance selon un quatrième mode de réalisation de l'invention.
[Fig. 7] les figures 7A à 7H sont des vues en plan de dessus d'un dispositif d'assistance selon le quatrième mode de réalisation de l'invention à différents états de verrouillage et déverrouillage des moyens de verrouillage.
[Fig. 8] la figure 8 est une vue éclatée en perspective d'une partie d'un dispositif d'assistance selon un cinquième mode de réalisation de l'invention.
[Fig. 9] les figures 9A à 9H sont des vues en coupe d'un dispositif d'assistance selon le cinquième mode de réalisation de l'invention à différents états de verrouillage et déverrouillage des moyens de verrouillage.
[Fig. 10] les figures 10A à 10H sont des vues en coupe d'un dispositif d'assistance selon un sixième mode de réalisation de l'invention à différents états de verrouillage et déverrouillage des moyens de verrouillage.
[Fig. 11] la figure 11 est une vue éclatée en perspective d'une partie d'un dispositif d'assistance selon un septième mode de réalisation de l'invention.
[Fig. 12] les figures 12A à 12H sont des vues en coupe d'un dispositif d'assistance selon le septième mode de réalisation de l'invention à différents états de verrouillage et déverrouillage des moyens de verrouillage.

### Description détaillée

Dans les modes de réalisation présentés ci-après, on décrit des dispositifs d'assistance à l'utilisation d'un dispositif de distribution de produit dans lesquels le dispositif de distribution est un spray nasal et le produit est un médicament, notamment un médicament antidouleur à base d'opioïdes. Il est possible de prévoir que le dispositif de distribution soit tout autre dispositif de distribution connu de l'homme du métier, par exemple un dispositif de distribution de produit liquide sous forme de goutte. On peut également prévoir que le produit soit tout autre produit compatible avec une utilisation avec un dispositif de distribution de produit, ce produit pouvant être un médicament ou non, par exemple un produit cosmétique. Dans la description qui suit, les éléments homologues entre les différents modes de réalisation se voient attribuer un numéro de référence dont seul le chiffre des centaines est modifié.

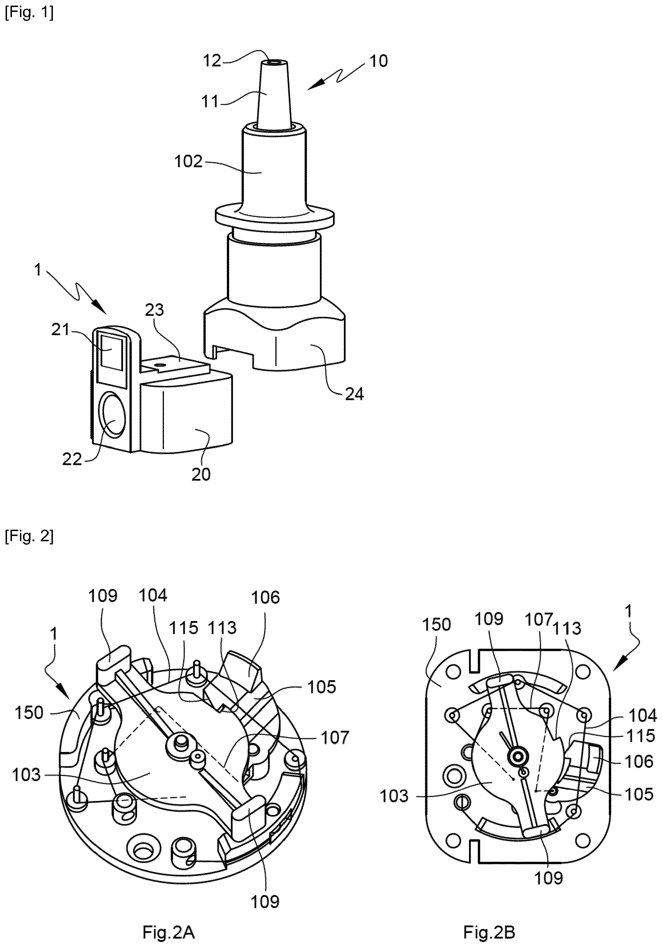

On a représenté sur les figures 1 à 3C un dispositif d'assistance 1 à l'utilisation d'un dispositif de distribution 10 de produit selon un premier mode de réalisation de l'invention.

Le dispositif de distribution 10 auquel est solidarisé le dispositif d'assistance 1 comprend une pompe 11 présentant un embout de distribution 12 et un réservoir (non représenté). Le dispositif de distribution 10 comprend en outre une partie mobile 102 formant un applicateur. Cet applicateur 102 est monté mobile à coulissement le long d'un axe longitudinal principal du dispositif de distribution 10 entre une position de repos, dans laquelle l'applicateur 102 est dans sa position la plus distale possible et donc la plus proche de l'embout de distribution 12, et une position d'activation qui est la position minimale que doit occuper l'applicateur 102 pour que la totalité de la dose de produit qu'il est prévu de distribuer lors d'un actionnement du dispositif de distribution 10 soit effectivement distribuée. Il peut s'agir de la position la plus proximale de l'applicateur 102, c'est-à-dire la plus éloignée possible de l'embout de distribution 12. On peut prévoir qu'il s'agisse d'une autre position prédéterminée, moins proximale, c'est-à-dire moins éloignée de l'embout de distribution 12. De même, il est possible que tout autre dispositif de distribution 10 soit utilisé en association avec le dispositif d'assistance 1 selon l'invention, à condition que son fonctionnement soit compatible avec celui du dispositif d'assistance 1. Ceci permettrait à ce que le dispositif d'assistance 1 constitue un dispositif réutilisable pouvant être assemblé et en interaction successivement avec différents dispositifs de distribution 10, de différents types ou non. Ces dispositifs de distribution 10 constituant alors des dispositifs de type "jetable" après chaque fin d'utilisation. Une fin d'utilisation pouvant se traduire par une fin de traitement, un réservoir vide ou à un niveau de remplissage insuffisant pour une distribution appropriée de produit.

Dans le présent mode de réalisation, le dispositif d'assistance 1 est en plusieurs parties. On peut toutefois prévoir que le dispositif d'assistance 1 soit réalisé en une seule partie. Une première partie est formée par un socle 20 réutilisable. Ce socle 20 comprend, entre autres, des moyens de signalement comprenant un écran 21 apte à afficher un signal visuel, un lecteur d'empreinte 22 et des composants électroniques (non représentés) utiles pour le contrôle et le suivi des différentes fonctions du dispositif d'assistance 1 qui seront détaillées ci-après. Le socle 20 comprend une paroi supérieure à partir de laquelle s'étend une broche 23. Le dispositif d'assistance comprend en outre des moyens de verrouillage 103 (voir figures 2A et 2B) compris dans une base 24. La base 24 comprend une paroi inférieure à partir de laquelle s'étend une broche complémentaire (non représentée) de forme complémentaire à celle de la broche 23 s'étendant à partir du socle 20 et apte à venir en prise avec celle-ci de manière à solidariser la base 24 et le socle 20 et à les mettre en communication électrique. Le dispositif d'assistance 1 comprend également des moyens de solidarisation (non représentés) au dispositif de distribution 10.

Les moyens de verrouillage 103 comprennent un élément ayant une forme générale de disque dont deux portions diamétralement opposées se prolongent en une protubérance qui s'étend dans un même plan que celui dans lequel s'étend le disque.

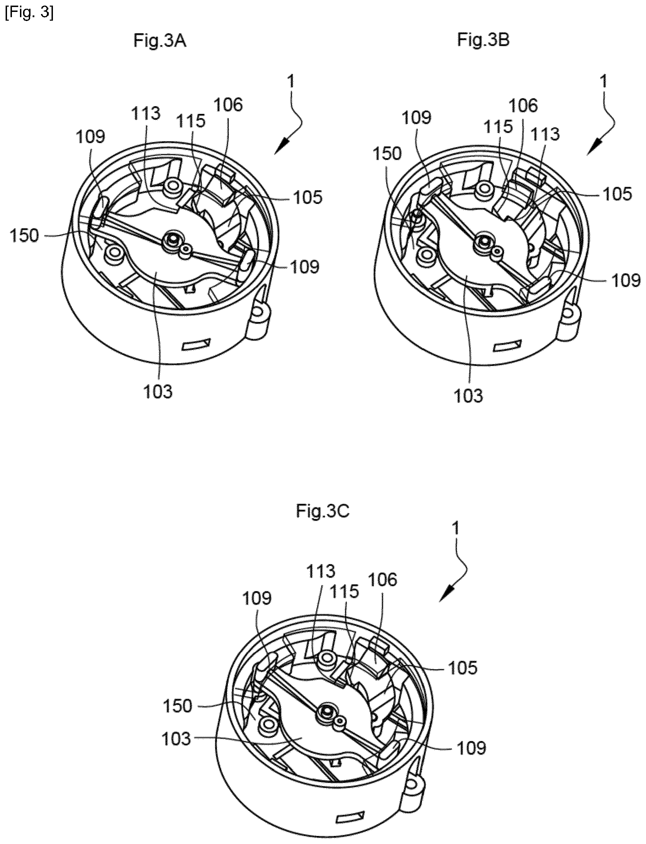

Les moyens de verrouillage 103 comprennent un plot 109 s'étendant perpendiculairement à partir de chacune des protubérances du disque. Ces plots 109 sont donc au nombre de deux. On peut prévoir qu'il y ait un nombre différent de protubérances s'étendant à partir du disque, par exemple entre une et dix, de préférence entre deux et quatre. On peut donc également prévoir qu'il y ait un nombre différent de plots 109 s'étendant à partir de chacune des protubérances, par exemple entre un et dix, de préférence entre deux et quatre. Les plots 109 sont de forme générale circulaire à section oblongue. On peut prévoir qu'ils aient une autre forme, par exemple une forme de section différente telle que carrée, rectangulaire, ovale, ronde ou parallélépipédique. Le disque des moyens de verrouillage 103 présente une encoche 113 ménagée au niveau d'une périphérie du disque. Le disque des moyens de verrouillage 103 est fixé sur un plateau 150 du dispositif d'assistance 1 et est monté mobile en rotation par rapport à celui-ci. Par conséquent, les plots 109 qui ne forment qu'une pièce avec le disque sont eux aussi mobile en rotation par rapport au dispositif d'assistance 1. Cette rotation se fait autour d'un axe passant par le centre du disque et perpendiculaire au plan dans lequel s'étend le disque. Les moyens de verrouillage 103 sont mobiles en rotation entre une position de verrouillage dans laquelle les deux plots 109 sont chacun respectivement en butée contre une butée du dispositif d'assistance 1 (voir figure 3A) et une position de déverrouillage dans laquelle les plots 109 des moyens de verrouillage sont à distance de la butée (voir figure 2A et 3B). Comme il sera décrit ci-après, en position de verrouillage, les moyens de verrouillage 103 empêchent la distribution de produit par le dispositif de distribution 10, et en position de déverrouillage, les moyens de verrouillage 103 n'empêchent pas la distribution de produit.

Le dispositif d'assistance 1 comprend en outre un actionneur 104 configuré pour faire passer les moyens de verrouillage 103 de la position de verrouillage à la position de déverrouillage (voir figures 2A et 2B). Dans le cas présent, l'actionneur 104 est un fil rétractable. Les fils rétractables sont bien connus de l'homme du métier et fonctionnent, lorsqu'ils sont traversés par un courant électrique, en se rétractant d'une longueur qui est fonction de l'intensité du courant électrique. Le fil rétractable 104 est fixé par une de ses extrémités au plateau 150 et par l'autre de ses extrémités au disque des moyens de verrouillage 103. Lorsqu'un courant parcourt le fil rétractable 104, ce dernier se rétracte. Le plateau 150 étant immobile, l'extrémité du fil rétractable 104 liée au plateau est elle aussi immobile alors que l'extrémité liée au disque des moyens de verrouillage 103, sous l'effet de la rétractation du fil 104, entraîne la rotation des moyens de verrouillage 103 de manière à les faire passer de la position de verrouillage à la position de déverrouillage. On peut prévoir que l'actionneur 104 soit un autre élément qu'un fil rétractable, par exemple un dispositif manuel, un solénoïde ou un électro-aimant.

Le dispositif d'assistance 1 comprend en outre des moyens de retour (non représentés) configurés pour faire passer les moyens de verrouillage 103 de la position de déverrouillage à la position de verrouillage. Dans le cas présent, les moyens de retour comprennent un ressort de torsion qui est contraint lors du passage des moyens de verrouillage 103 de la position de verrouillage à la position de déverrouillage. Ainsi, ces moyens de retour appliquent sur les moyens de verrouillage 103 une force tendant à les ramener dans la position de verrouillage.

Le dispositif d'assistance 1 comprend en outre des moyens de blocage 105 configurés pour bloquer les moyens de verrouillage 103 en position de déverrouillage. Dans le cas présent, les moyens de blocage 105 ont une forme générale de virgule et sont fixés au plateau 150 de manière mobile en rotation par une des extrémités de la virgule. Les moyens de blocage 105 comprennent une face interne dirigée en direction des moyens de verrouillage 103 qui est courbée et dont la courbure est complémentaire d'une courbure du disque des moyens de verrouillage 103 à laquelle la courbure des moyens de blocage 105 fait face. Cette face interne des moyens de blocage 105 comprend en outre une encoche complémentaire 115 apte à venir en prise avec l'encoche 113 des moyens de verrouillage 103 de manière à maintenir ces derniers en position de déverrouillage malgré la force de retour exercée par les moyens de retour.

Le dispositif d'assistance 1 comprend en outre des premiers moyens de désengagement 106 des moyens de blocage 105 configurés pour être déclenchés lors de l'activation du dispositif de distribution 10. Les premiers moyens de désengagement 106 sont formés par une protubérance s'étendant à partir d'une face supérieure des moyens de blocage 105 et ne formant qu'une pièce avec ceux-ci. Les premiers moyens de désengagement 106 présentent une face interne en regard des moyens de verrouillage 103 et formant une rampe. Ces premiers moyens de désengagement 106 sont aptes à désengager les moyens de verrouillages 103 des moyens de blocage 105 de manière à permettre aux moyens de retour d'exercer leur effet et donc de déplacer les moyens de verrouillage 103 vers leur position de verrouillage. Les premiers moyens de désengagement 106 sont ainsi agencés de sorte que lorsqu'on fait parcourir leur rampe de haut en bas, c'est-à-dire depuis son extrémité libre vers son extrémité fixée aux moyens de blocage 105, par un élément appliquant une force suffisante, les premiers moyens de désengagement 106 sont déplacés et entraînent avec eux les moyens de blocage 105 en rotation de manière à ce que l'encoche 113 et l'encoche complémentaire 115 des moyens de verrouillage 103 et des moyens de blocage 105 ne soient plus en prise et donc que les moyens de verrouillage 103 retournent en position de verrouillage sous l'effet des moyens de retour. Lors de l'activation du dispositif de distribution 10, une partie de l'applicateur 102 applique une force sur les premiers moyens de désengagement 106 et parcoure leur rampe de haut en bas de manière à réaliser le désengagement mentionné ci-dessus.

Le dispositif d'assistance 1 comprend également des seconds moyens de désengagement 107 des moyens de blocage 105 configurés pour être déclenchés indépendamment de l'activation du dispositif de distribution 10 du produit. Dans le cas présent, ces seconds moyens de désengagement 107 comprennent un fil rétractable. Ce fil rétractable 107 est identique dans sa nature au fil rétractable 104 utilisé en tant qu'actionneur pour faire passer les moyens de verrouillage 103 de la position de verrouillage à la position de déverrouillage. Une extrémité du fil rétractable 107 est fixée au plateau 150 et son autre extrémité est fixée aux moyens de blocage 105. Lorsqu'on fait parcourir un courant électrique dans ce fil rétractable 107, il se rétracte. Puisque son extrémité fixée au plateau 150 est immobile, elle tire sur son autre extrémité qui est fixée aux moyens de blocage 105 et entraîne ceux-ci en rotation de manière à ce que, lorsque les moyens de verrouillage 103 et de blocage 105 sont en prises via l'encoche 113 et l'encoche complémentaire 115, les moyens de blocage 105 s'éloignent des moyens de verrouillage 103, notamment au niveau de l'encoche 113 et de l'encoche complémentaire 115 de manière à ce qu'elles ne soient plus en prises. Les moyens de verrouillage 103 sont alors entraînés en rotation par les moyens de retour qui les déplacent vers la position de verrouillage. L'actionnement du fil rétractable 107 dépend uniquement du courant qui le traverse et est donc indépendant d'un actionnement du dispositif de distribution 10. On peut prévoir que ces seconds moyens de désengagement 107 ne consistent pas en un fil rétractable mais en un autre élément apte à déplacer les moyens de blocage 105, par exemple un moteur et/ou un ou plusieurs aimants et/ou un ou plusieurs électro-aimants.

Le dispositif d'assistance 1 comprend également un pion (non représenté) présentant au moins un cran complémentaire de forme complémentaire à au moins un cran présent sur une partie de l'applicateur 102. Ces au moins un cran et un cran complémentaire sont agencés de manière à empêcher un actionnement partiel du dispositif de distribution 10. Ainsi, à partir du moment où l'applicateur 102 a parcouru une première course prédéterminée suffisante pour initier la distribution de produit, et tant que l'applicateur 102 n'a pas parcouru une deuxième course prédéterminée, correspondant au moins à une course nécessaire pour la délivrance d'une dose complète de produit, la partie mobile du dispositif de distribution 10 via l'applicateur 102 ne peut pas retrouver sa position de repos tant qu'au moins la deuxième course prédéterminée n'a pas été parcourue par l'applicateur 102.

Dans une configuration préférentielle, le dispositif d'assistance 1 comprend une pièce d'assemblage (non représentée) formant un cache recouvrant les moyens de verrouillage 103 et permettant un renforcement de l'assemblage de l'ensemble du système de verrouillage et de déverrouillage en plus de sa stabilité. Cette pièce d'assemblage serait assemblée avec les moyens de verrouillage afin d'être solidaire de ces derniers et de limiter leur accès limité à tout utilisateur.

Nous décrivons maintenant un mode de fonctionnement du dispositif d'assistance 1 selon ce premier mode de réalisation.

À l'état de repos, le dispositif de distribution 10 est dans sa position de repos et les moyens de verrouillage 103 sont en position de verrouillage (voir figure 3A). En position de verrouillage, les plots 109 des moyens de verrouillage 103 sont en regard de protubérances présentes sur l'applicateur 102. Ces protubérances de l'applicateur 102 viennent en butée contre les plots 109 lorsque l'utilisateur tente d'utiliser le dispositif de distribution 10 sans l'avoir déverrouillé, ce qui empêche l'actionnement du dispositif de distribution 10. Lorsque l'utilisateur souhaite utiliser le dispositif, il place son doigt sur le lecteur d'empreinte 22 afin de le déverrouiller, étant entendu que son empreinte digitale a préalablement été enregistrée de manière à ce qu'il soit reconnu en tant qu'utilisateur autorisé par le dispositif d'assistance 1. Lorsque l'empreinte digitale de l'utilisateur est authentifiée, des moyens de contrôle électroniques via les composants électroniques présents dans le socle 20 commandent la production d'un courant électrique et son parcours dans le fil rétractable 104 formant l'actionneur de manière à ce que celui-ci se rétracte. En se rétractant, le fil rétractable 104 permet le passage des moyens de verrouillage 103 de la position de verrouillage (figure 3A) à la position de déverrouillage (figure 3B) par rotation de ces moyens de verrouillage 103 dans le sens horaire par rapport aux figures 2A à 3C. Au cours de la rotation des moyens de verrouillage 103, l'encoche complémentaire 115 des moyens de blocage 105 parcourt le pourtour extérieur du disque des moyens de verrouillage 103 jusqu'à venir en prise avec l'encoche 113 des moyens de verrouillage 103 (voir figure 3B). De cette façon, les moyens de verrouillage 103 sont bloqués en position de verrouillage par les moyens de blocage 105. En position de déverrouillage, les protubérances de l'applicateur 102 ne sont plus en regard des plots 109 des moyens de verrouillage 103 qui ont été déplacés au cours de la rotation des moyens de verrouillage 103. Ainsi, ces protubérances ne butent plus contre les plots 109 lorsqu'un utilisateur tente d'actionner le dispositif de distribution 10. L'actionnement du dispositif de distribution 10 est donc possible.

Deux options sont alors possibles : soit l'utilisateur actionne le dispositif de distribution 10, soit il ne l'actionne pas.

Dans le cas où l'utilisateur actionne le dispositif de distribution 10, il déplace l'applicateur 102 en direction proximale, c'est-à-dire vers le bas par référence à la figure 1. Au cours de l'actionnement, l'applicateur 102 est déplacé vers la position d'actionnement du dispositif de distribution 10. Lors de ce déplacement, une portion de l'applicateur 102 (non représentée) applique une pression sur les premiers moyens de désengagement 106 en parcourant leur rampe. Le dispositif d'assistance 1 est agencé de manière à ce que le parcours de la rampe des premiers moyens de désengagement 106 par la portion de l'applicateur 102 ne permette le désengagement des moyens de blocage 105 et des moyens de verrouillage 103 qu'une fois que la position d'activation a été atteinte, ceci afin d'éviter que le dispositif de distribution 10 ne se reverrouille alors qu'une dose complète n'a pas été délivrée. Ainsi, lorsque la portion de l'applicateur 102 a parcouru la course prédéterminée, les moyens de verrouillage 103 et les moyens de blocage 105 sont désengagés l'un de l'autre et les moyens de retour rappellent les moyens de verrouillage 103 en position de verrouillage (voir figure 3C).

Il est possible d'intégrer au dispositif d'assistance 1 des moyens de détection permettant de détecter le nombre de doses distribuées par le dispositif. Ces moyens de détection peuvent comprendre au moins une tige de contact destinée à être en butée avec les moyens de blocage 105 après que ces derniers aient été désengagés des moyens de verrouillage 103 par les premiers moyens de désengagement 106, une fois la position d'activation atteinte. Le contact entre la tige de contact et les moyens de blocage 105 permet de traduire l'information suivante : la position d'activation est atteinte donc une dose de produit a été distribuée et les moyens de blocage 105 ont été désengagés. Cette information confirmant la bonne distribution d'une dose de produit, peut être transmise aux moyens de contrôle électroniques pour le suivi et le contrôle du dispositif de distribution et de la distribution du produit.

Dans le cas où l'utilisateur n'actionne pas le dispositif de distribution 10, soit parce que le déverrouillage a eu lieu accidentellement, soit parce qu'il ne désire finalement pas obtenir une dose de produit, il est alors nécessaire de pouvoir reverrouiller le dispositif, afin notamment d'éviter qu'une personne non autorisée l'utilise, sans pour autant actionner le dispositif de distribution 10, afin notamment de ne pas gaspiller du produit. Pour cela, on commande, par exemple grâce à un bouton et via les moyens de contrôle électroniques présents dans le socle 20, le parcours d'un courant électrique dans les seconds moyens de désengagement 107 formés par le fil rétractable. En se rétractant, ce fil rétractable 107 entraîne les moyens de blocage 105 en rotation de manière à ce que l'encoche 115 de ceux-ci ne soit plus en prise avec l'encoche 113 des moyens de verrouillage 103. Les moyens de verrouillages 103 ainsi libérés sont ramenés en position de verrouillage par les moyens de retour sans qu'il y ait besoin d'actionner le dispositif de distribution 10. On peut prévoir, alternativement ou cumulativement, que la commande du désengagement des moyens de verrouillage 103 soit effectuée autrement. Par exemple, on peut prévoir que le dispositif d'assistance 1 comprend un chronomètre qui est lancé lorsque les moyens de verrouillage 103 passent de la position de verrouillage à la position de déverrouillage et que les moyens de contrôle électroniques via les composants électroniques présents dans le socle 20 sont configurés pour commander le parcours d'un courant électrique dans le fil rétractable 107 lorsque le chronomètre atteint une valeur prédéterminée. On peut également prévoir, alternativement ou cumulativement, que le dispositif d'assistance 1 et/ou le dispositif de distribution 10 comprend un capteur d'inclinaison du dispositif d'assistance 1 et/ou du dispositif de distribution 10 et que les moyens de contrôle électroniques sont configurés pour commander le parcours d'un courant électrique dans le fil rétractable 107 lorsque l'inclinaison du dispositif d'assistance 1 et/ou du dispositif de distribution 10 mesurée par le capteur d'inclinaison atteint ou dépasse un seuil prédéterminé.

Dans les modes de réalisation présentés ci-après, les éléments homologues entre les modes de réalisation sont nommés de la même façon que dans le premier mode de réalisation et ont la même fonction malgré leur forme ou leur nature qui peut être complètement différente.

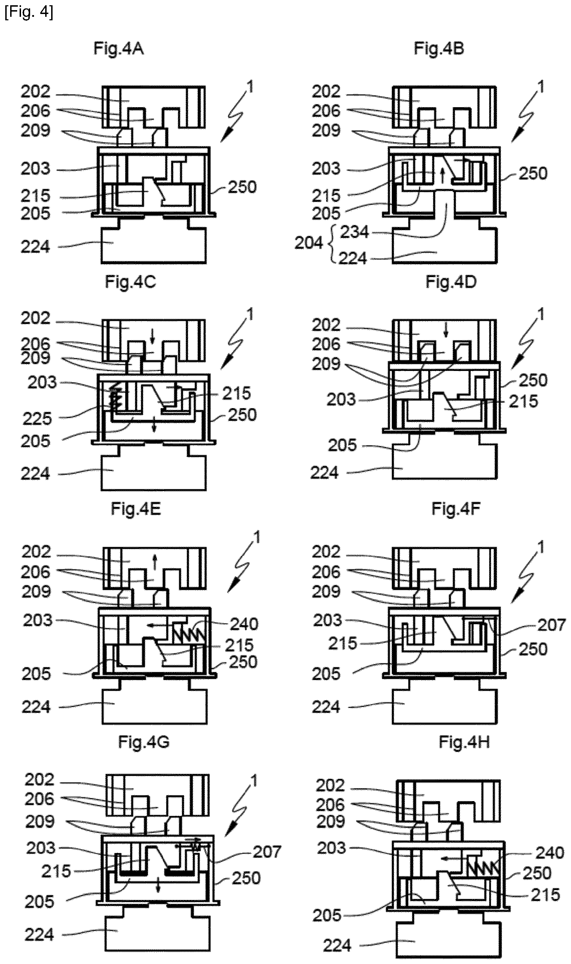

On a représenté sur les figures 4A à 4H un dispositif d'assistance 1 à l'utilisation d'un dispositif de distribution 10 de produit selon un deuxième mode de réalisation de l'invention.

Dans ce mode de réalisation, les moyens de verrouillage 203 ont une forme générale parallélépipédique et comprennent des plots 209 qui s'étendent à partir de deux extrémités opposées. Les plots 209 ont également une forme générale d'un parallélépipède mais présentent chacun un chanfrein formant une rampe. Les rampes de tous les plots 209 sont orientées dans la même direction et avec un même angle d'inclinaison (voir figures 4A à 4H). Dans le cas présent, les plots 209 sont au nombre de quatre, deux au niveau de chacune des deux extrémités opposées des moyens de verrouillage 203. On peut prévoir un nombre différent de plots 209, par exemple entre deux et vingt, de préférence entre quatre et huit, voire entre quatre et six. Les moyens de verrouillage 203 sont montés sur le dispositif d'assistance 1 à coulissement horizontal, c'est-à-dire selon la direction d'un axe perpendiculaire à un axe longitudinal principal du dispositif d'assistance 1. Les moyens de verrouillage 203 présentent des ouvertures au niveau d'une paroi inférieure du parallélépipède qu'ils forment.

Les moyens de blocage 205 sont également de forme générale parallélépipédique avec des dimensions globales supérieures à celles des moyens de verrouillage 203 de manière à ce que les moyens de blocage 205 soient aptes à contenir au moins en partie les moyens de verrouillage 203. Les moyens de blocage 205 comprennent deux protubérances formant des clips 215 s'étendant à partir d'une surface supérieure principale des moyens de blocage 205. Les clips 215 sont mobiles en translation verticale, c'est-à-dire le long d'un axe longitudinal principal du dispositif d'assistance 1 mais sont immobiles en translation horizontale, c'est-à-dire le long d'un axe perpendiculaire à l'axe longitudinal principal du dispositif d'assistance 1. En position de repos, les clips 215 ne sont pas en prise avec les moyens de verrouillage 203 et des extrémités d'une paroi inférieure des moyens de verrouillage 203 sont en appui contre une rampe inclinée de chacun des clips 215 (voir figure 4A). En position de blocage, les clips 215 sont en prise avec les moyens de verrouillage 203 et empêchent ceux-ci de retourner en position de verrouillage. Un ressort 225 est comprimé lors du passage des moyens de blocage 205 de la position de repos à la position de blocage et applique donc une force tendant à ramener les moyens de blocage 205 en position de repos. Par souci de clarté, ce ressort 225 est uniquement visible sur la figure 4C.

Dans le présent mode de réalisation, l'actionneur 204 comprend une base 224 et un pion 234 mobile en translation verticale par rapport à cette base 224 entre une position rentrée dans laquelle le pion 234 s'étend entièrement ou presque entièrement à l'intérieur de la base 224 et une position sortie dans laquelle le pion 234 forme une saillie au niveau d'une paroi supérieure de la base 224. Le pion 234, en position sortie, présente un axe longitudinal principal qui est perpendiculaire au plan dans lequel s'étend la paroi supérieure de la base 234 à partir de laquelle le pion 234 s'étend.

Une portion de l'extrémité proximale de l'applicateur 202 forme des créneaux comprenant des crêtes et des creux. Les creux sont configurés pour pouvoir accueillir les plots 209 des moyens de verrouillage 203. Une partie des crêtes de l'applicateur 202 forme les premiers moyens de désengagement 206. Ainsi, les moyens de verrouillage 203 sont mobiles à coulissement horizontal entre une position de verrouillage (voir figure 4A) dans laquelle les sommets des plots 209 sont en regard d'une des crêtes de l'extrémité proximale de l'applicateur 202 et une position de déverrouillage (voir figure 4B) dans laquelle les sommets des plots 209 sont en regard des creux de l'extrémité proximale de l'applicateur 202 tandis que les rampes des plots 209 sont, elles, en regard des crêtes de l'extrémité proximale de l'applicateur 202.

Les moyens de retour 240 configurés pour faire passer les moyens de verrouillage 203 de la position de déverrouillage à la position de verrouillage sont ici représentés par un ressort en compression (représenté uniquement sur les figures 4E et 4H dans un souci de clarté des figures). Ce ressort 240 est comprimé lors du passage des moyens de verrouillage 203 de la position de verrouillage à la position de déverrouillage et applique donc une force sur ceux-ci tendant à les ramener en position de verrouillage.

Les seconds moyens de désengagement 207 comprennent ici un fil rétractable dont l'une des extrémités est fixée à un boîtier 250 contenant les moyens de verrouillage 203 et les moyens de blocage 205. L'autre extrémité du fil rétractable 207 est fixée aux moyens de verrouillage 203. Ainsi, le fil rétractable 207 est apte, en se rétractant sous l'effet d'un courant électrique, à entraîner les moyens de verrouillages 203 en translation horizontale en direction de la position de déverrouillage. Par soucis de clarté, les seconds moyens de désengagement 207 sont uniquement représentés sur les figures 4F et 4G.

Nous décrivons maintenant un mode de fonctionnement du dispositif d'assistance 1 selon ce deuxième mode de réalisation.

À l'état de repos, la partie mobile du dispositif de distribution 10 via l'applicateur 202 est dans sa position de repos, les moyens de verrouillage 203 sont en position de verrouillage et les moyens de blocage 205 sont en position de repos (voir figure 4A). Comme indiqué précédemment, en position de verrouillage, les plots 209 des moyens de verrouillage 203 sont en regard de crêtes de l'extrémité proximale de l'applicateur 202. Ces crêtes de l'applicateur 202 viennent en butée contre les sommets des plots 209 lorsque l'utilisateur tente d'utiliser le dispositif de distribution 10 sans l'avoir déverrouillé ce qui empêche l'actionnement du dispositif de distribution 10. Lorsque l'utilisateur souhaite utiliser le dispositif de distribution 10, il place son doigt sur le lecteur d'empreinte 22 afin de le déverrouiller, étant entendu que son empreinte digitale a préalablement été enregistrée de manière à ce qu'il soit reconnu en tant qu'utilisateur autorisé par le dispositif d'assistance 1. Lorsque l'empreinte digitale de l'utilisateur est authentifiée, les moyens de contrôle électroniques via les composants électroniques présents dans le socle 20 commandent à l'actionneur 204 la sortie du pion 234 hors de la base 224. Au cours de sa sortie, le pion 234 entraîne en translation verticale, c'est-à-dire le long de l'axe longitudinal principal du dispositif d'assistance 1, en direction distale, c'est-à-dire en direction du dispositif de distribution 10, les moyens de blocage 205. Lors de cette translation verticale des moyens de blocage 205, la paroi inférieure des moyens de verrouillage 203 qui est en appui contre la rampe des clips 215 parcourt cette rampe. Puisque les moyens de blocage 205 ne sont pas mobiles en translation horizontale, les moyens de verrouillage 203, au fur et à mesure du parcours de la rampe des clips 215 par leur paroi inférieure, sont entraînés en translation horizontale de manière à passer en position de déverrouillage et, en fin de parcours de la rampe, les clips 215 viennent en prise avec la paroi inférieure des moyens de verrouillage 203 (voir figure 4B). De cette façon, les moyens de verrouillage 203 sont bloqués en position de déverrouillage par les moyens de blocage 205. En position de déverrouillage, les crêtes de l'extrémité de l'applicateur 202 ne sont plus en regard des sommets des plots 209 des moyens de verrouillage 203 mais en regard des rampes de ces plots 209. Ainsi, ces protubérances ne butent plus contre les sommets des plots 209 lorsqu'un utilisateur tente d'actionner le dispositif de distribution 10 mais parcourent la rampe de chaque plot 209 (voir figure 4C). L'actionnement du dispositif de distribution 10 est donc possible.

Deux options sont alors envisageables : soit l'utilisateur actionne le dispositif de distribution 10, soit il ne l'actionne pas.

Dans le cas où l'utilisateur actionne le dispositif de distribution 10, il déplace l'actionneur 202 en direction proximale, c'est-à-dire vers le bas par référence aux figures 4A à 4H. Au cours de l'actionnement, l'applicateur 202 est déplacé vers la position d'actionnement du dispositif de distribution 10. Lors de ce déplacement, les crêtes de l'extrémité de l'applicateur 202 parcourent les rampes inclinées des plots 209 (voir figure 4C). L'applicateur 202 n'étant pas mobile en translation horizontale, les moyens de verrouillage 203 sont eux entraînés en translation horizontale de manière à ce qu'ils ne soient plus en prise avec les clips 215 et à ce que les plots 209 se logent, au fur et à mesure de la descente de l'applicateur 202 dans les creux de celui-ci. Les moyens de blocage 205 n'étant plus en prise avec les moyens de verrouillage 203, ils sont rappelés en position de repos par le ressort 225 (voir figures 4A et 4D). Une fois l'actionnement terminé, l'applicateur 202 remonte de manière à être en position de repos et les moyens de retour 240 font passer les moyens de verrouillage 203 en position de verrouillage (voir figure 4E). La situation de départ est donc retrouvée, c'est-à-dire que les moyens de verrouillage 203 sont en position de verrouillage et le dispositif de distribution 10 et les moyens de blocage 205 sont en position de repos.

Dans le cas où l'utilisateur n'actionne pas le dispositif de distribution 10, soit parce que le déverrouillage a eu lieu accidentellement, soit parce qu'il ne désire finalement pas obtenir une dose de produit, il est alors nécessaire de pouvoir reverrouiller le dispositif de distribution 10, afin notamment d'éviter qu'une personne non autorisée l'utilise, sans pour autant actionner le dispositif de distribution 10, afin notamment de ne pas gaspiller du produit. Pour cela, on commande, par exemple grâce à un bouton et via les moyens de contrôle électroniques présents dans le socle 20, le parcours d'un courant électrique dans les seconds moyens de désengagement 207 formés par le fil rétractable (voir figure 4F). En se rétractant, ce fil rétractable 207 entraîne en translation horizontale les moyens de verrouillage de manière à ce qu'ils ne soient plus en prise avec les moyens de blocage 205 (voir figure 4G). Ainsi, le ressort 225 entraîne les moyens de blocage 205 en translation verticale vers le bas de manière à les ramener en position de repos et les moyens de retour 240 formés par le ressort entraînent les moyens de verrouillage 203 en translation horizontale jusqu'à la position de verrouillage (voir figure 4H) sans qu'il y ait besoin d'actionner le dispositif de distribution 10. Comme indiqué précédemment on peut prévoir, alternativement ou cumulativement, que la commande du désengagement des moyens de verrouillage 203 soit effectuée autrement.

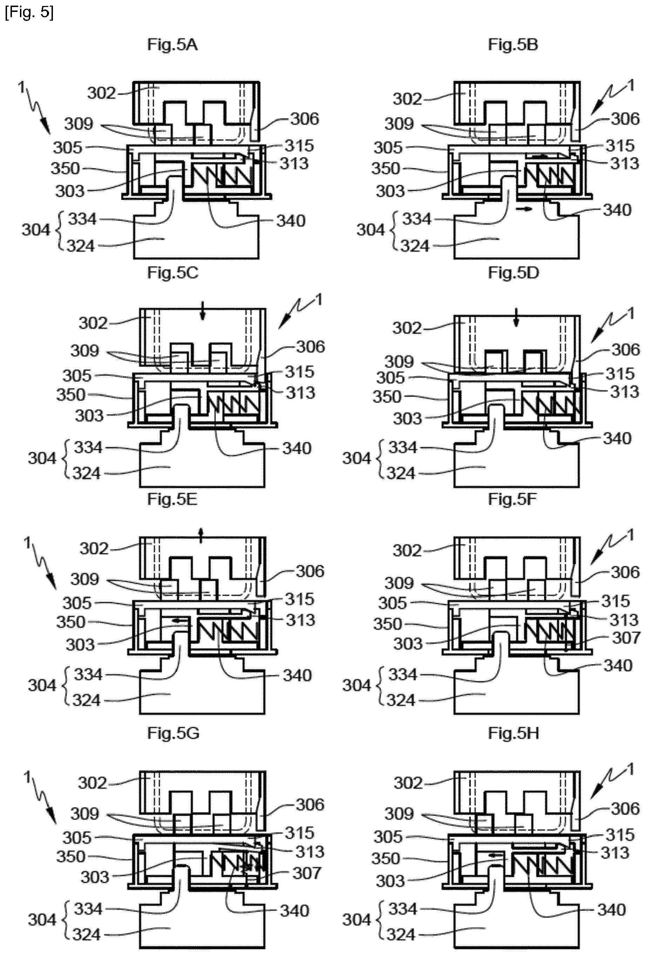

On a représenté sur les figures 5A à 5H un dispositif d'assistance 1 à l'utilisation d'un dispositif de distribution 10 de produit selon un troisième mode de réalisation de l'invention.

Dans ce mode de réalisation, les moyens de verrouillage 303 ont une forme générale parallélépipédique et comprennent des plots 309 qui s'étendent à partir de deux extrémités opposées. Les plots 309 ont également une forme générale d'un parallélépipède. Dans le cas présent, les plots 309 sont au nombre de quatre, deux au niveau de chacune de deux extrémités opposées des moyens de verrouillage 303. On peut prévoir un nombre différent de plot 309, par exemple entre deux et vingt, de préférence entre quatre et huit, voire entre quatre et six. Les moyens de verrouillage 303 sont montés sur le dispositif d'assistance 1 à coulissement horizontal, c'est-à-dire selon la direction d'un axe perpendiculaire à l'axe longitudinal principal du dispositif d'assistance 1. Les moyens de verrouillage 303 présentent des ouvertures au niveau d'une paroi inférieure du parallélépipède qu'ils forment. Les moyens de verrouillage 303 comprennent une projection latérale. Au niveau d'une extrémité libre de cette projection latérale s'étend un ergot 313.

Les moyens de blocage 305 ont une forme générale de disque. On peut prévoir que les moyens de blocage 305 ont toute autre forme compatible avec une utilisation du dispositif d'assistance 1. Un ergot complémentaire 315 s'étend à partir d'une partie d'une portion périphérique des moyens de blocage 305 en direction de la projection latérale des moyens de verrouillage 303. Les moyens de blocage 305 sont montés immobiles par rapport au dispositif d'assistance 1. En position de repos, l'ergot 313 des moyens de verrouillage 303 et l'ergot complémentaire 315 des moyens de blocage 305 ne sont pas en prise et sont à distance l'un de l'autre (voir figure 5A). En position de blocage, l'ergot 313 des moyens de verrouillage 303 est en butée contre l'ergot complémentaire 315 des moyens de blocage 305 de manière à ce que les moyens de blocage 305 empêchent une translation horizontale des moyens de verrouillage 303 en direction de la position de verrouillage (voir figure 5B).

Les moyens de retour 340 configurés pour faire passer les moyens de verrouillage 303 de la position de déverrouillage à la position de verrouillage sont ici représentés par un ressort en compression. Ce ressort 340 est comprimé lors du passage des moyens de verrouillage 303 de la position de verrouillage à la position de déverrouillage et applique donc une force sur ceux-ci tendant à les ramener en position de verrouillage.

Dans le présent mode de réalisation, l'actionneur 304 comprend une base 324 et un pion 334 mobile en translation horizontale par rapport à cette base 324 entre une position de repos (voir figure 5A) et une position d'actionnement (voir figure 5B). Le pion 334 forme une saillie au niveau d'une paroi supérieure de la base 324 et présente un axe longitudinal principal qui est perpendiculaire au plan dans lequel s'étend la paroi supérieure de la base 324 à partir de laquelle le pion 334 s'étend. En position de repos, le pion 334 s'étend à l'intérieur des moyens de verrouillage 303 à travers une ouverture dans leur paroi inférieure et est en appui contre une paroi interne des moyens de verrouillage 303 (voir figure 5A). Le pion 334 est apte à entraîner les moyens de verrouillage 303 en translation grâce à l'appui contre cette paroi interne et permet ainsi le passage de la position de verrouillage vers la position de déverrouillage (voir figure 5B).

L'applicateur 302 comprend une protubérance formant les premiers moyens de désengagement 306. Cette protubérance est formée par un prolongement de la paroi de l'applicateur au niveau de son extrémité proximale. Cette protubérance 306 est apte, lorsque les moyens de verrouillage 303 sont bloqués en position de déverrouillage tel que représenté à la figure 5B, à venir appliquer une force sur l'ergot 313 des moyens de verrouillage 303 de manière à désengager la prise de celui-ci avec l'ergot complémentaire 315 des moyens de blocage 305 (voir figures 5C et 5D).

Une portion de l'extrémité proximale de l'applicateur 302 forme des créneaux comprenant des crêtes et des creux. Les creux sont configurés pour pouvoir accueillir les plots 309 des moyens de verrouillage 303. Ainsi, les moyens de verrouillage 303 sont mobiles à coulissement horizontal entre une position de verrouillage (voir figure 5A) dans laquelle les sommets des plots 309 sont chacun en regard d'une des crêtes de l'extrémité proximale de l'applicateur 302 et une position de déverrouillage (voir figure 5B) dans laquelle les sommets des plots 309 sont en regard chacun d'un des creux de l'extrémité proximale de l'applicateur 302.

Les seconds moyens de désengagement 307 comprennent ici un fil rétractable dont l'une des extrémités est fixée à un boîtier 350 contenant les moyens de verrouillage 303 et les moyens de blocage 305. L'autre extrémité du fil rétractable 307 est fixée à la projection latérale des moyens de verrouillage 303. Ainsi, le fil rétractable 307 est apte, en se rétractant sous l'effet d'un courant électrique, à déformer cette projection latérale de manière à ce que l'ergot 313 des moyens de verrouillage 303 ne soient plus en prise avec l'ergot complémentaire 315 des moyens de blocage 305. Par souci de clarté, les seconds moyens de désengagement 307 sont uniquement représentés sur les figures 5F et 5G.

Nous décrivons maintenant un mode de fonctionnement du dispositif d'assistance 1 selon ce troisième mode de réalisation.

À l'état de repos, le dispositif de distribution 10 est dans sa position de repos, les moyens de verrouillage 303 sont en position de verrouillage et les moyens de blocage 305 sont en position de repos (voir figure 5A). Comme indiqué précédemment, en position de verrouillage, les plots 309 des moyens de verrouillage 303 sont en regard de crêtes de l'extrémité proximale de l'applicateur 302. Ces crêtes de l'applicateur 302 viennent en butée contre les sommets des plots 309 lorsque l'utilisateur tente d'utiliser le dispositif de distribution 10 sans l'avoir déverrouillé, ce qui empêche donc l'actionnement du dispositif de distribution 10. Lorsque l'utilisateur souhaite utiliser le dispositif de distribution 10, il place son doigt sur le lecteur d'empreinte 22 afin de le déverrouiller de la même manière que pour les précédents modes de réalisation. Lorsque l'empreinte digitale de l'utilisateur est authentifiée, les moyens de contrôle électroniques via les composants électroniques présents dans le socle 20 commandent à l'actionneur 304 une translation horizontale du pion 334 de sa position de repos vers sa position d'actionnement. Au cours de cette translation, le pion 334, qui est en appui contre la paroi interne des moyens de verrouillage 303, entraîne ceux-ci en translation horizontale depuis leur position de verrouillage vers leur position de déverrouillage (voir figures 5A et 5B). L'ergot 313 des moyens de verrouillages 303 présente une rampe en direction de l'ergot 315 des moyens de blocage 305. L'ergot complémentaire 315 des moyens de blocage 305 présente une rampe complémentaire en direction de l'ergot 313 des moyens de verrouillages 303. Lors de la translation des moyens de verrouillage 303, la rampe de l'ergot 313 des moyens de verrouillage 303 parcourt celle de l'ergot complémentaire 315 des moyens de blocage 305 qui, elle, est fixe par rapport au dispositif d'assistance 1, de sorte que la projection latérale des moyens de verrouillage 303 se déforme élastiquement progressivement vers le bas au fur et à mesure que les moyens de verrouillage 303 translatent en direction de la position de déverrouillage sous l'effet du pion 334. Une fois que la totalité de la rampe de l'ergot complémentaire 315 a été parcourue, la projection latérale reprend sa conformation initiale de sorte que l'ergot 313 et l'ergot complémentaire 315 sont en appui l'un contre l'autre de manière à ce que les moyens de blocage 305 empêchent les moyens de verrouillage 303 de se déplacer en direction de la position de verrouillage (voir figure 5B). Les moyens de verrouillage 303 sont ainsi bloqués en position de déverrouillage.

En position de déverrouillage, les crêtes de l'extrémité de l'applicateur 302 ne sont plus en regard des sommets des plots 309 des moyens de verrouillage 303. Ainsi, les crêtes de l'extrémité de l'applicateur 302 ne viennent plus en butée contre les sommets des plots 309 lorsqu'un utilisateur tente d'actionner le dispositif de distribution 10. L'actionnement du dispositif de distribution 10 est donc possible (voir figure 5C).

Deux options sont alors envisageables : soit l'utilisateur actionne le dispositif de distribution 10, soit il ne l'actionne pas.

Dans le cas où l'utilisateur actionne le dispositif de distribution 10, il déplace l'actionneur 302 en direction proximale, c'est-à-dire vers le bas par référence aux figures 5A à 5H. Au cours de l'actionnement, l'applicateur 302 est déplacé vers la position d'actionnement du dispositif de distribution 10. Les plots 309 se logent, au fur et à mesure de la descente de l'applicateur 302 dans les creux de celui-ci. Au bout d'une course prédéterminée, les premiers moyens de désengagement 306 atteignent l'ergot 313 des moyens de verrouillage 303 et leur appliquent une force de manière à déformer élastiquement la projection latérale des moyens de verrouillage 303 (voir figure 5D) et ainsi libérer l'ergot 313 des moyens de verrouillage 303 de l'ergot complémentaire 315 des moyens de blocage 305. Lorsque l'ergot 313 et l'ergot complémentaire 315 ne sont plus en appui l'un contre l'autre, le ressort des moyens de retour 340 exerce son action de manière à entraîner un coulissement horizontal des moyens de verrouillage 303 jusqu'à leur position de verrouillage (voir figure 5E). La situation de départ est donc retrouvée, c'est-à-dire que les moyens de verrouillage 303 sont en position de verrouillage et le dispositif de distribution 10 et les moyens de blocage 305 sont en position de repos.

Dans le cas où l'utilisateur n'actionne pas le dispositif de distribution 10, soit parce que le déverrouillage a eu lieu accidentellement, soit parce qu'il ne désire finalement pas obtenir une dose de produit, il est alors nécessaire de pouvoir reverrouiller le dispositif de distribution 10, afin notamment d'éviter qu'une personne non autorisée l'utilise, sans pour autant actionner le dispositif de distribution 10, afin notamment de ne pas gaspiller du produit. Pour cela, on commande, par exemple grâce à un bouton et via les moyens de contrôle électroniques présents dans le socle 20, le parcours d'un courant électrique dans les seconds moyens de désengagement 307 formés par le fil rétractable (voir figure 5F). En se rétractant, ce fil rétractable 307 déforme élastiquement la projection latérale des moyens de verrouillage 303 vers le bas de manière à désengager l'ergot 313 et l'ergot complémentaire 315 l'un de l'autre (voir figure 5G). Ainsi, les moyens de retour 340 entraînent les moyens de verrouillage 303 en translation horizontale jusqu'à la position de verrouillage (voir figure 5H) sans qu'il y ait besoin d'actionner le dispositif de distribution 10. Comme indiqué précédemment on peut prévoir, alternativement ou cumulativement, que la commande du désengagement des moyens de verrouillage 303 soit effectuée autrement.

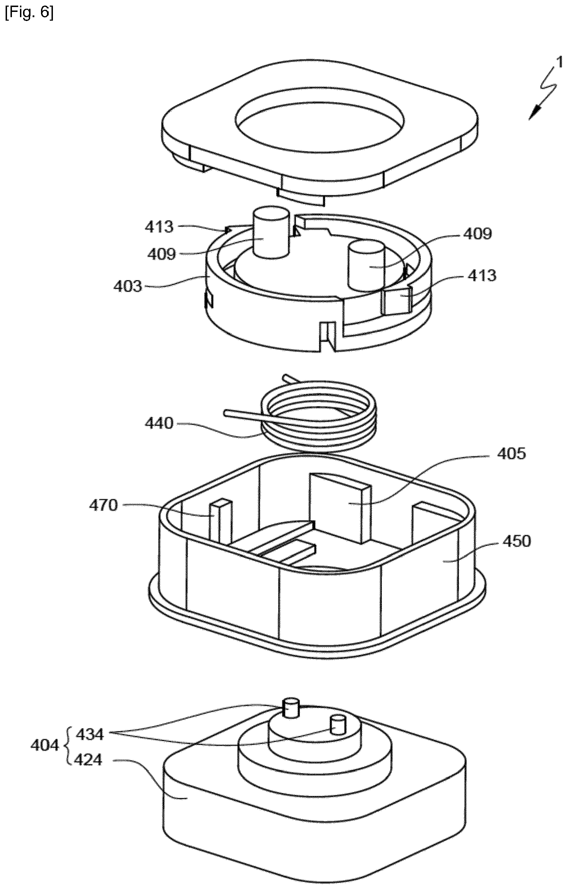

On a représenté sur les figures 6 à 7H un dispositif d'assistance 1 à l'utilisation d'un dispositif de distribution 10 de produit selon un quatrième mode de réalisation de l'invention.

Dans ce mode de réalisation, les moyens de verrouillage 403 ont une forme générale cylindrique circulaire et comprennent des plots 409 qui s'étendent à partir d'une paroi supérieure des moyens de verrouillage 403. Les plots 409 ont également une forme générale cylindrique circulaire (voir figure 6). Dans le cas présent, les plots 409 sont au nombre de deux et sont diamétralement opposés. On peut prévoir un nombre différent de plots 409, par exemple entre deux et vingt, de préférence entre deux et huit, voire entre deux et six. Les moyens de verrouillage 403 sont montés sur le dispositif d'assistance 1 en rotation autour d'un axe longitudinal principal du dispositif d'assistance 1. Les moyens de verrouillage 403 comprennent deux projections latérales circonférentielles s'étendant chacune sur une partie du pourtour des moyens de verrouillage 403. Au niveau d'une extrémité libre de chacune de ces projections latérales s'étend un ergot 413.

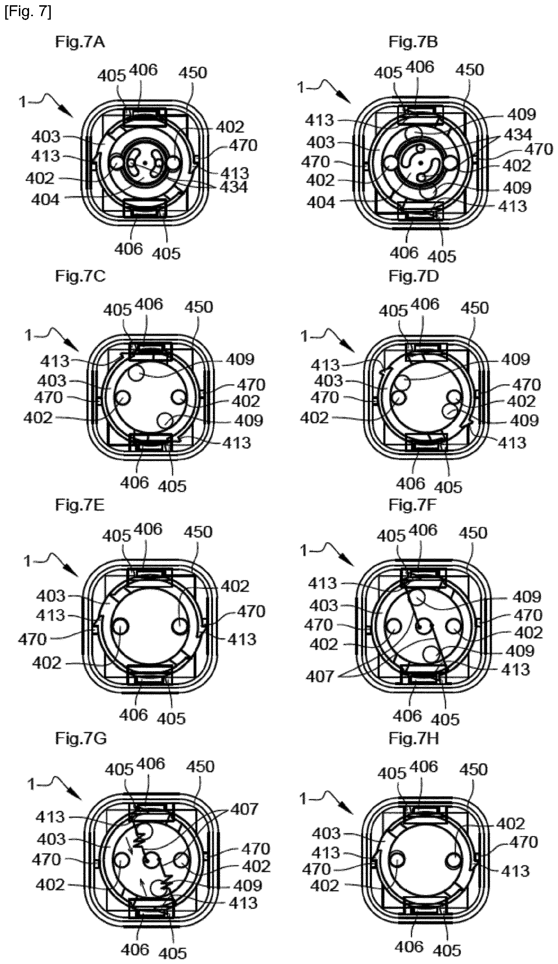

Le dispositif d'assistance 1 comprend un boîtier 450 apte à contenir les moyens de verrouillage 403. Les moyens de blocage 405 sont formés par des reliefs s'étendant à partir d'une face interne d'une paroi latérale du boîtier 450. Les moyens de blocage 405 sont donc immobiles par rapport au dispositif d'assistance 1 dans cette configuration. Le boitier 450 comprend en outre deux reliefs 470 s'étendant respectivement sur deux faces internes des parois latérales du boitier 450, ces parois étant différentes de celles à partir desquelles s'étendent les moyens de blocage 450. En position de repos, l'ergot 413 des moyens de verrouillage 403 et le relief des moyens de blocage 405 ne sont pas en prises et sont à distance l'un de l'autre (voir figure 7A). Chaque ergot 413 des moyens de verrouillage 403 vient alors en butée contre un des reliefs 470 (voir figure 7A). En position de blocage, les ergots 413 des moyens de verrouillage 403 sont chacun en butée contre une face plane d'un des reliefs des moyens de blocage 405 de manière à ce que les moyens de blocage 405 empêchent une rotation des moyens de verrouillage 403 en direction de la position de verrouillage (voir figure 7B).

Les moyens de retour 440 configurés pour faire passer les moyens de verrouillage 403 de la position de déverrouillage à la position de verrouillage sont ici représentés par un ressort en torsion. Ce ressort 440 est comprimé lors du passage des moyens de verrouillage 403 de la position de verrouillage à la position de déverrouillage et applique donc une force sur ceux-ci tendant à les ramener en position de verrouillage.

Dans le présent mode de réalisation, l'actionneur 404 comprend une base 424 et deux pions 434 mobiles en rotation par rapport à cette base 424 autour de l'axe longitudinal principal du dispositif d'assistance 1 entre une position de repos (voir figure 7A) et une position d'actionnement (voir figure 7B). Les pions 434 forment une saillie au niveau d'une paroi supérieure de la base 424 et présentent chacun un axe longitudinal principal qui est perpendiculaire au plan dans lequel s'étend la paroi supérieure de la base 434 à partir de laquelle ils s'étendent. Les pions 434 s'étendent à l'intérieur des moyens de verrouillage 403 à travers une ouverture dans leur paroi inférieure. Les pions 434 sont aptes à entraîner les moyens de verrouillage 403 en rotation autour de l'axe longitudinal principal de l'applicateur 402 et permettent ainsi le passage de la position de verrouillage vers la position de déverrouillage.

L'applicateur 402 comprend deux protubérances formant les premiers moyens de désengagement 406. Ces protubérances sont formées par un prolongement de la paroi de l'applicateur 402 au niveau de son extrémité proximale. Ces protubérances 406 sont aptes, lorsque les moyens de verrouillage 403 sont bloqués en position de déverrouillage tel que représenté à la figure 7B à venir appliquer une force sur l'ergot 413 des moyens de verrouillage 403 de manière à désengager la prise des ergots 413 avec les reliefs des moyens de blocage 405 en déformant élastiquement les projections latérales des moyens de verrouillage 403 (voir figure 7C).

L'applicateur 402 comprend des plots de forme générale cylindrique circulaire et ayant sensiblement le même diamètre que les plots 409 des moyens de verrouillage 403. Les moyens de verrouillage 403 sont mobiles en rotation entre une position de verrouillage (voir figure 7A) dans laquelle les sommets des plots 409 sont chacun en regard du sommet d'un des plots respectifs de l'applicateur 402 et une position de déverrouillage (voir figure 7B) dans laquelle les sommets des plots 409 des moyens de verrouillage 403 ne sont pas en regard du sommet des plots de l'applicateur 402.

Les seconds moyens de désengagement 407 comprennent ici deux fils rétractables dont l'une des extrémités de chacun de ces fils rétractables 407 est fixée à une portion centrale des moyens de verrouillage 403. L'autre extrémité de chacun de ces fils rétractables 407 est fixée à une des projections latérales des moyens de verrouillage 403 respectives des moyens de verrouillage 403. Ainsi, les fils rétractables 407 sont aptes, en se rétractant sous l'effet d'un courant électrique, à déformer élastiquement ces projections latérales de manière à ce que les ergots 413 des moyens de verrouillage 403 ne soient plus en prise avec les reliefs des moyens de blocage 405. Par souci de clarté, les seconds moyens de désengagement 407 sont uniquement représentés sur les figures 7F et 7G.

Nous décrivons maintenant un mode de fonctionnement du dispositif d'assistance 1 selon ce quatrième mode de réalisation.

À l'état de repos, le dispositif de distribution 10 est dans sa position de repos et les moyens de verrouillage 403 sont en position de verrouillage (voir figure 7A). Comme indiqué précédemment, en position de verrouillage, les plots 409 des moyens de verrouillage 403 sont en regard de plots de l'applicateur 402. Ces plots de l'applicateur 402 viennent en butée contre les sommets des plots 409 lorsque l'utilisateur tente d'utiliser le dispositif de distribution 10 sans l'avoir déverrouillé ce qui empêche l'actionnement du dispositif de distribution 10. Lorsque l'utilisateur souhaite utiliser le dispositif de distribution 10, il place son doigt sur le lecteur d'empreinte 22 afin de le déverrouiller de la même manière que pour les précédents modes de réalisation. Lorsque l'empreinte digitale de l'utilisateur est authentifiée, les moyens de contrôle électroniques via les composants électroniques présents dans le socle 20 commandent à l'actionneur 404 une rotation des pions 434 de sa position de repos vers sa position d'actionnement. Au cours de cette rotation, les pions 434 entraînent les moyens de verrouillage 403 depuis leur position de verrouillage vers leur position de déverrouillage (voir figures 7A et 7B). Les ergots 413 des moyens de verrouillage 403 présentent chacun une rampe en direction d'un des reliefs respectifs des moyens de blocage 405. Lors de la rotation des moyens de verrouillage 403, les rampes respectives des ergots 413 des moyens de verrouillage 403 sont parcourues chacune respectivement par un des reliefs des moyens de blocage 405. Les reliefs des moyens de blocage 405 étant fixes par rapport au dispositif d'assistance 1, les projections latérales des moyens de verrouillage 403 se déforment élastiquement progressivement vers le centre des moyens de verrouillage 403 au fur et à mesure que les moyens de verrouillage 403 tournent sous l'effet de la rotation des pions 434. Une fois que la totalité de chaque rampe des ergots 413 a été parcourue, chaque projection latérale des moyens de verrouillage 403 reprend sa conformation initiale de sorte que les ergots 413 des moyens de verrouillage 403 et les reliefs des moyens de blocage 405 sont en appui l'un contre l'autre de sorte que les moyens de blocage 405 empêchent les moyens de verrouillage 403 de se déplacer en direction de la position de verrouillage sous l'action du ressort de torsion 440 (voir figure 7B). Les moyens de verrouillage 403 sont ainsi bloqués en position de déverrouillage.

En position de déverrouillage, les plots de l'applicateur 402 ne sont plus en regard des sommets des plots 409 des moyens de verrouillage 403. Ainsi, ces plots ne butent plus contre ces sommets lorsqu'un utilisateur tente d'actionner le dispositif de distribution 10. L'actionnement du dispositif de distribution 10 est donc possible.

Deux options sont alors envisageables : soit l'utilisateur actionne le dispositif de distribution 10, soit il ne l'actionne pas.

Dans le cas où l'utilisateur actionne le dispositif de distribution 10, il déplace l'actionneur 402 en direction proximale, c'est-à-dire vers le bas par référence à la figure 6. Au cours de l'actionnement, l'applicateur 402 est déplacé vers la position d'actionnement du dispositif de distribution 10. Au bout d'une course prédéterminée, les premiers moyens de désengagement 406 atteignent les ergots 413 des moyens de verrouillage 403 et leur appliquent une force de manière à déformer élastiquement les projections latérales des moyens de verrouillage 403 (voir figure 7C). Lorsque chaque ergot 413 des moyens de verrouillage 403 et chaque relief des moyens de blocage 405 ne sont plus en appui l'un contre l'autre, le ressort de torsion des moyens de retour 440 exerce son action de manière à entraîner en rotation les moyens de verrouillage 403 jusqu'à leur position de verrouillage (voir figure 7E). La situation de départ est donc retrouvée, c'est-à-dire que les moyens de verrouillage 403 sont en position de verrouillage et le dispositif de distribution 10 est en position de repos.

Dans le cas où l'utilisateur n'actionne pas le dispositif de distribution 10, soit parce que le déverrouillage a eu lieu accidentellement, soit parce qu'il ne désire finalement pas obtenir une dose de produit, il est alors nécessaire de pouvoir reverrouiller le dispositif de distribution 10, afin notamment d'éviter qu'une personne non autorisée l'utilise, sans pour autant actionner le dispositif de distribution 10, afin notamment de ne pas gaspiller du produit. Pour cela, on commande, par exemple grâce à un bouton et via les moyens de contrôle électroniques présents dans le socle 20, le parcours d'un courant électrique dans les seconds moyens de désengagement 407 formés par les fils rétractable (voir figure 7F). En se rétractant, ces fils rétractables 407 déforment élastiquement les projections latérales des moyens de verrouillage 403 vers le centre des moyens de verrouillage 403 de manière à désengager les ergots 413 des moyens de verrouillage 403 et les reliefs des moyens de blocage 405 l'un de l'autre (voir figure 7G). Ainsi, les moyens de retour 440 entraînent les moyens de verrouillage 403 en rotation jusqu'à la position de verrouillage (voir figure 7H) sans qu'il y ait besoin d'actionner le dispositif de distribution 10. Comme indiqué précédemment on peut prévoir, alternativement ou cumulativement, que la commande du désengagement des moyens de verrouillage 403 soit effectuée autrement.

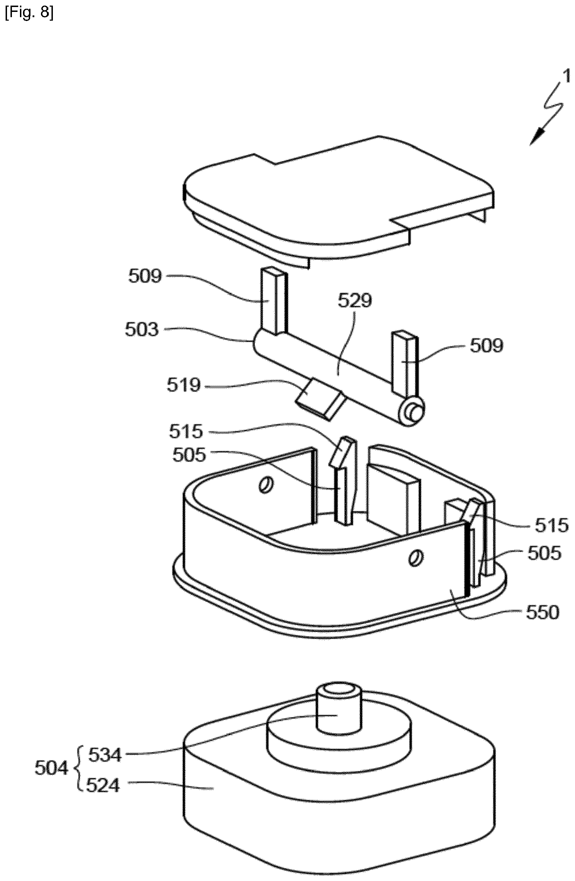

On a représenté sur les figures 8 à 9H un dispositif d'assistance 1 à l'utilisation d'un dispositif de distribution 10 de produit selon un cinquième mode de réalisation de l'invention.

Dans ce mode de réalisation, les moyens de verrouillage 503 ont une forme générale cylindrique circulaire à partir de laquelle des plots 509 s'étendent à partir de chacune des extrémités d'un cylindre 529 perpendiculairement à un axe longitudinal principal du cylindre 529 (voir figure 8). Les plots 509 ont une forme générale parallélépipédique. Dans le cas présent, les plots 509 sont au nombre de deux. On peut prévoir un nombre différent de plots 509, par exemple entre deux et vingt, de préférence entre deux et huit, voire entre deux et six. Les moyens de verrouillage 503 comprennent en outre une protubérance 519 s'étendant radialement à partir d'une portion médiane du cylindre 529. Les moyens de verrouillage 503 sont montés sur le dispositif d'assistance 1 en rotation autour d'un axe perpendiculaire à l'axe longitudinal principal du dispositif d'assistance 1.

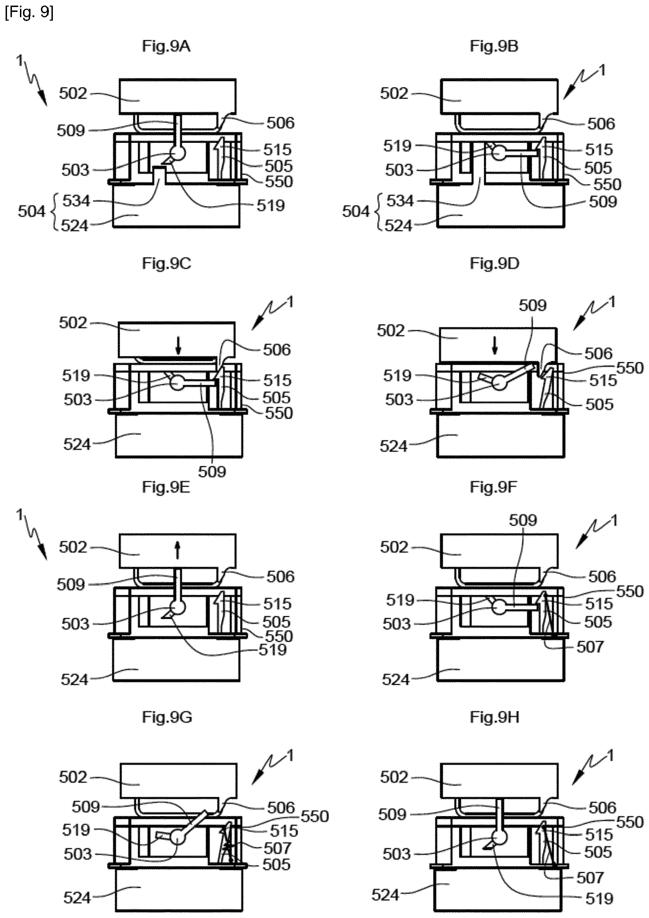

Le dispositif d'assistance 1 comprend un boîtier 550 apte à contenir les moyens de verrouillage 503. Les moyens de blocage 505 comprennent deux protubérances s'étendant à partir d'une face interne d'une paroi inférieure du boîtier 550 et présentant chacune un ergot 515 au niveau de leur extrémité libre. En position de repos, les plots 509 des moyens de verrouillage 503 et les ergots 515 des moyens de blocage 505 ne sont pas en prises et sont à distance les uns des autres (voir figure 9A). En position de blocage, les plots 509 des moyens de verrouillage 503 sont en butée contre un des ergots 515 respectifs des moyens de blocage 505 de manière à ce que les moyens de blocage 505 empêchent une rotation des moyens de verrouillage 503 en direction de la position de verrouillage (voir figure 9B).

Les moyens de retour (non représentés) configurés pour faire passer les moyens de verrouillage 503 de la position de déverrouillage à la position de verrouillage comprennent ici un ressort de torsion. Ce ressort est comprimé lors du passage des moyens de verrouillage 503 de la position de verrouillage à la position de déverrouillage et applique donc une force sur ceux-ci tendant à les ramener en position de verrouillage.

Dans le présent mode de réalisation, l'actionneur 504 comprend une base 524 et un pion 534 mobile en translation verticale par rapport à cette base 524 entre une position de repos (voir figure 9A) et une position d'actionnement (voir figure 9B). Le pion 534 forme une saillie au niveau d'une paroi supérieure de la base 524 et présente un axe longitudinal principal qui est perpendiculaire au plan dans lequel s'étend la paroi supérieure de la base 534 à partir de laquelle il s'étend. Le pion 534 est apte à entraîner les moyens de verrouillage 503 en rotation autour de leur axe longitudinal principal et permet ainsi le passage de la position de verrouillage vers la position de déverrouillage. Pour cela, le pion 534 pousse sur la protubérance 519 des moyens de verrouillage 503 (voir figure 9B). On peut prévoir que l'actionneur 504 soit tout autre élément apte à remplir cette fonction. On peut par exemple prévoir que l'actionneur 504 soit un fil rétractable apte, lorsqu'il se rétracte, à entraîner la rotation des moyens de verrouillage 503 de la position de verrouillage vers la position de déverrouillage.

L'applicateur 502 comprend deux protubérances formant les premiers moyens de désengagement 506. Ces protubérances 506 sont formées par un prolongement de la paroi de l'applicateur au niveau de son extrémité proximale. Ces protubérances 506 sont aptes, lorsque les moyens de verrouillage 503 sont bloqués en position de déverrouillage par les moyens de blocage 505 tel que représenté à la figure 9B à venir appliquer une force sur chacun des ergots 515 des moyens de blocage 505 de manière à désengager la prise de ceux-ci avec les plots 509 des moyens de verrouillage 503 en déformant élastiquement les moyens de blocage 505 (voir figures 9C et 9D).

Les moyens de verrouillage 503 sont mobiles en rotation entre une position de verrouillage (voir figure 9A) dans laquelle les sommets des plots 509 sont chacun en regard d'un sommet d'une extrémité proximale de l'applicateur 502 et une position de déverrouillage (voir figure 9B) dans laquelle les sommets des plots 509 ne sont pas en regard d'un sommet de l'extrémité proximale de l'applicateur 502.

Les seconds moyens de désengagement 507 comprennent ici deux fils rétractables dont, pour chacun de ces fils rétractables 507, l'une des extrémités est fixée au boîtier 550. L'autre extrémité de chacun de ces fils rétractables 507 est fixée à un des ergots 515 respectifs des moyens de blocage 505. Ainsi, les fils rétractables 507 sont aptes, en se rétractant sous l'effet d'un courant électrique, à déformer élastiquement ces moyens de blocage 505 de manière à ce que les plots 509 des moyens de verrouillage 503 ne soient plus en prise avec les ergots 515 des moyens de blocage 505. Par souci de clarté, les seconds moyens de désengagement 507 sont uniquement représentés sur les figures 9F à 9H.

Nous décrivons maintenant un mode de fonctionnement du dispositif d'assistance 1 selon ce cinquième mode de réalisation.

À l'état de repos, le dispositif de distribution 10 est dans sa position de repos et les moyens de verrouillage 503 sont en position de verrouillage (voir figure 9A). Comme indiqué précédemment, en position de verrouillage, les plots 509 des moyens de verrouillage 503 sont chacun en regard d'un sommet de l'extrémité proximale de l'applicateur 502. Les sommets de l'applicateur 502 viennent en butée contre les sommets des plots 509 lorsque l'utilisateur tente d'utiliser le dispositif de distribution 10 sans l'avoir déverrouillé ce qui empêche l'actionnement du dispositif de distribution 10. Lorsque l'utilisateur souhaite utiliser le dispositif de distribution 10, il place son doigt sur le lecteur d'empreinte 22 afin de le déverrouiller de la même manière que pour les précédents modes de réalisation. Lorsque l'empreinte digitale de l'utilisateur est authentifiée, les moyens de contrôle électroniques via les composants électroniques présents dans le socle 20 commandent à l'actionneur 504 un passage du pion 534 de sa position de repos dans la base 524 vers sa position d'actionnement dans laquelle il fait saillie en dehors de la base 524. Au cours de cette sortie, le pion 534 entraîne la protubérance 519 des moyens de verrouillage 503, et donc les moyens de verrouillage 503 eux-mêmes, depuis leur position de verrouillage vers leur position de déverrouillage (voir figures 9A et 9B). Les ergots 515 des moyens de blocage 505 présentent une rampe en direction des plots 509. Chacun des plots 509 des moyens de verrouillage 503 parcourt une des rampes respectives des ergots 515 lors de la rotation des moyens de verrouillage 503. Au fur et à mesure que les plots 509 parcourent leur rampe respective, les moyens de blocage 505 sont déformés élastiquement. Une fois que la totalité de chaque rampe des ergots 515 a été parcourue, les moyens de blocage 505 reprennent leur conformation initiale de sorte que les plots 509 et les ergots 515 sont en appui l'un contre l'autre de manière à ce que les moyens de blocage 505 empêchent les moyens de verrouillage 503 de se déplacer en direction de la position de verrouillage sous l'action du ressort de torsion (voir figure 9B). Les moyens de verrouillage 503 sont ainsi bloqués en position de déverrouillage.

En position de déverrouillage, les sommets de l'extrémité proximale de l'applicateur 502 ne sont plus en regard des sommets des plots 509 des moyens de verrouillage 503. Ainsi, ces sommets de l'extrémité proximale de l'applicateur 502 ne butent plus contre les sommets des plots 509 lorsqu'un utilisateur tente d'actionner le dispositif de distribution 10. L'actionnement du dispositif de distribution 10 est donc possible.

Deux options sont alors envisageables : soit l'utilisateur actionne le dispositif de distribution 10, soit il ne l'actionne pas.

Dans le cas où l'utilisateur actionne le dispositif de distribution 10, il déplace l'actionneur 502 en direction proximale, c'est-à-dire vers le bas par référence aux figures 8 à 9H. Au cours de l'actionnement, l'applicateur 502 est déplacé vers la position d'actionnement du dispositif de distribution 10. Au bout d'une course prédéterminée, les premiers moyens de désengagement 506 atteignent l'ergot 515 des moyens de blocage 505 et leur applique une force de manière à déformer élastiquement les moyens de blocage 505 (voir figures 9C et 9D). Lorsque les plots 509 et les ergots 515 ne sont plus en appui l'un contre l'autre, le ressort de torsion des moyens de retour exerce son action de manière à entraîner en rotation les moyens de verrouillage 503 jusqu'à leur position de verrouillage (voir figure 9E). La situation de départ est donc retrouvée, c'est-à-dire que les moyens de verrouillage 503 sont en position de verrouillage et le dispositif de distribution 10 est en position de repos.

Dans le cas où l'utilisateur n'actionne pas le dispositif de distribution 10, soit parce que le déverrouillage a eu lieu accidentellement, soit parce qu'il ne désire finalement pas obtenir une dose de produit, il est alors nécessaire de pouvoir reverrouiller le dispositif de distribution 10, afin notamment d'éviter qu'une personne non autorisée l'utilise, sans pour autant actionner le dispositif de distribution 10, afin notamment de ne pas gaspiller du produit. Pour cela, on commande, par exemple grâce à un bouton et via les moyens de contrôle électroniques présents dans le socle 20, le parcours d'un courant électrique dans les seconds moyens de désengagement 507 formés par les fils rétractable (voir figure 9F). En se rétractant, ces fils rétractables 507 déforment élastiquement les moyens de blocage 505 vers l'extérieur du dispositif d'assistance 1 de manière à désengager les plots 509 et ergots 515 les uns des autres (voir figure 9G). Ainsi, les moyens de retour entraînent les moyens de verrouillage 503 en rotation jusqu'à la position de verrouillage (voir figure 9H) sans qu'il y ait besoin d'actionner le dispositif de distribution 10. Comme indiqué précédemment on peut prévoir, alternativement ou cumulativement, que la commande du désengagement des moyens de verrouillage 503 soit effectuée autrement.

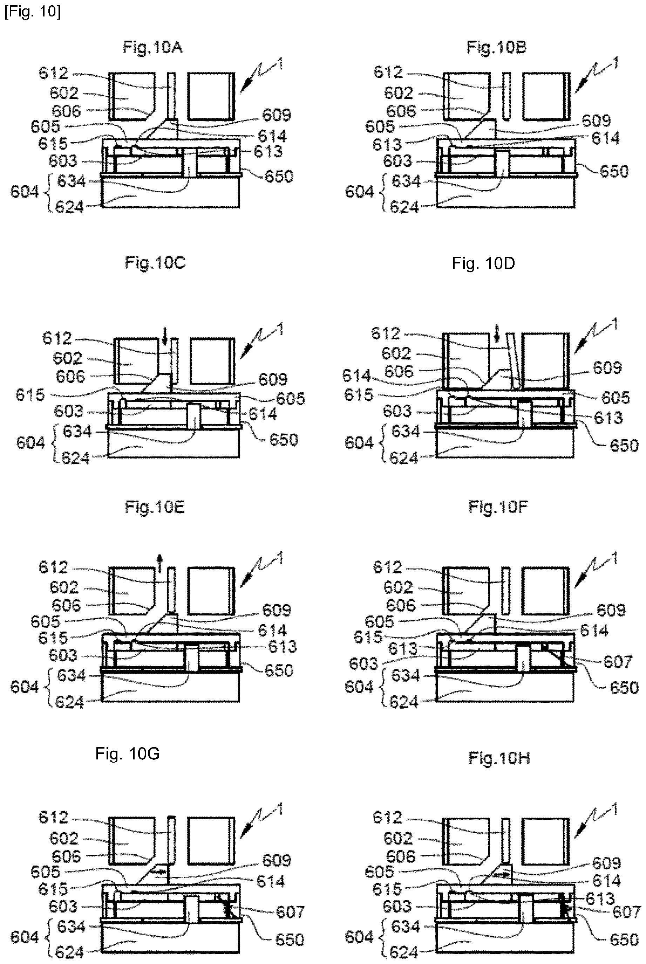

On a représenté sur les figures 10A à 10H un dispositif d'assistance 1 à l'utilisation d'un dispositif de distribution 10 de produit selon un sixième mode de réalisation de l'invention.

Dans ce mode de réalisation, les moyens de verrouillage 603 ont une forme générale d'un parallélépipède plat et comprennent des plots 609 qui s'étendent perpendiculairement vers le haut à partir d'une paroi supérieure des moyens de verrouillage 603. Les plots 609 ont également une forme générale d'un parallélépipède mais dont une arête a été biseautée de manière à former une rampe. Dans le cas présent, les plots 609 sont au nombre de deux et s'étendent sur deux côtés opposés des moyens de verrouillage 603. On peut prévoir un nombre différent de plots 609, par exemple entre deux et vingt, de préférence entre deux et huit, voire entre deux et six. Les moyens de verrouillage 603 sont montés sur le dispositif d'assistance 1 mobiles en translation horizontale. Les moyens de verrouillage 603 comprennent deux fentes longitudinales délimitant une lame dont une extrémité libre se termine par un ergot 613 sous la forme d'un bourrelet.

Le dispositif d'assistance 1 comprend un boîtier 650 apte à contenir les moyens de verrouillage 603. Le boîtier 650 comprend une paroi supérieure formant les moyens de blocage 605. Une face interne de la paroi supérieure 605 présente une première et une seconde cavités 614, 615 chacune apte à accueillir l'ergot 613. La première cavité 614 est configurée de manière à loger l'ergot 613 lorsque les moyens de verrouillage 603 sont en position de verrouillage et à bloquer ainsi les moyens de verrouillage 603 dans cette position. La seconde cavité 615 est configurée de manière à loger l'ergot 613 lorsque les moyens de verrouillage 603 sont en position de déverrouillage et à bloquer ainsi les moyens de verrouillage 603 dans cette position de déverrouillage.

Dans le présent mode de réalisation, l'actionneur 604 comprend une base 624 et un pion 634 mobile en translation horizontale par rapport à la base 624 entre une position de repos (voir figure 10A) et une position d'actionnement (voir figure 10B). Le pion 634 forme une saillie au niveau d'une paroi supérieure de la base 624 et présente un axe longitudinal principal qui est perpendiculaire au plan dans lequel s'étend la paroi supérieure de la base 634 à partir de laquelle il s'étend. Le pion 634 s'étend à l'intérieur des moyens de verrouillage 603 à travers une ouverture dans leur paroi inférieure. Le pion 634 est apte alors à entraîner les moyens de verrouillage 603 en translation horizontale et permet ainsi le passage de la position de verrouillage vers la position de déverrouillage.

L'applicateur 602 comprend deux fentes présentes chacune sur une paroi latérale de l'applicateur 602, ces deux parois étant opposées dans le cas présent. Dans chaque fente est présente une lame 612. La fente est délimitée par deux extrémités proximale de l'applicateur 602, un de ces extrémités étant biseauté et formant ainsi les premiers moyens de désengagement 606. Ces premiers moyens de désengagement 606 sont aptes, lorsque les moyens de verrouillage 603 sont bloqués en position de déverrouillage tel que représenté à la figure 10B à venir appliquer une force sur les plots 609 des moyens de verrouillage 603 de manière à désengager l'ergot 613 des moyens de verrouillage 603 de la seconde cavité 615 des moyens de blocage 605 (voir figure 10C).

Les moyens de verrouillage 603 sont mobiles en translation horizontale entre une position de verrouillage (voir figure 10A) dans laquelle les sommets des plots 609 sont chacun en regard du sommet d'une des lames 612 respectives de l'applicateur 602 et une position de déverrouillage (voir figure 10B) dans laquelle les sommets des plots 609 ne sont pas en regard du sommet des lames 612 de l'applicateur 602 et où le biseau des premiers moyens de désengagement 606 est plus proche de la rampe des plots 609 qu'en position de verrouillage.

Les seconds moyens de désengagement 607 comprennent ici un fil rétractable dont l'une des extrémités est fixée au boîtier 650 et l'autre extrémité est fixée aux moyens de verrouillage 603. Ainsi, le fil rétractable 607 est apte, en se rétractant sous l'effet d'un courant électrique, à entraîner en translation horizontale les moyens de verrouillage 603 vers la position de verrouillage. La force développée par le fil rétractable 607 est pour cela supérieure aux forces de frottement maintenant l'ergot 613 dans la seconde cavité 615 des moyens de blocage 605. Par souci de clarté, les seconds moyens de désengagement 607 sont uniquement représentés sur les figures 10F à 10H.

Nous décrivons maintenant un mode de fonctionnement du dispositif d'assistance 1 selon ce sixième mode de réalisation.

À l'état de repos, le dispositif de distribution 10 est dans sa position de repos et les moyens de verrouillage 603 sont en position de verrouillage (voir figure 10A). Comme indiqué précédemment, en position de verrouillage, les plots 609 des moyens de verrouillage 603 sont en regard des sommets des lames 612 de l'applicateur 602. Ces lames 612 viennent en butée contre les sommets des plots 609 lorsque l'utilisateur tente d'utiliser le dispositif de distribution 10 sans l'avoir déverrouillé ce qui empêche l'actionnement du dispositif de distribution 10. Lorsque l'utilisateur souhaite utiliser le dispositif de distribution 10, il place son doigt sur le lecteur d'empreinte 22 afin de le déverrouiller de la même manière que pour les précédents modes de réalisation. Lorsque l'empreinte digitale de l'utilisateur est authentifiée, les moyens de contrôle électroniques via les composants électroniques présents dans le socle 20 commandent à l'actionneur 604 une translation horizontale du pion 634 de sa position de repos vers sa position d'actionnement. Au cours de cette translation, le pion 634, qui est en appui contre une paroi interne des moyens de verrouillage 603, entraîne ceux-ci en translation horizontale depuis leur position de verrouillage vers leur position de déverrouillage (voir figures 10A et 10B). Lors de cette translation, la force appliquée par le pion 634 est suffisante pour faire sortir l'ergot 613 de la première cavité 614 des moyens de blocage 605. À la fin de la translation, l'ergot 613 vient se loger dans la seconde cavité 615 des moyens de blocage 605 de manière à ce que ces derniers bloquent les moyens de verrouillage 603 en position de déverrouillage (voir figure 10B).

En position de déverrouillage, les sommets des lames 612 de l'applicateur 602 ne sont plus en regard des sommets des plots 609 des moyens de verrouillage 603. Ainsi, ces lames 612 ne butent plus contre les sommets des plots 609 lorsqu'un utilisateur tente d'actionner le dispositif de distribution 10. L'actionnement du dispositif de distribution 10 est donc possible (voir figure 10B).

Deux options sont alors envisageables : soit l'utilisateur actionne le dispositif de distribution 10, soit il ne l'actionne pas.

Dans le cas où l'utilisateur actionne le dispositif de distribution 10, il déplace l'actionneur 602 en direction proximale, c'est-à-dire vers le bas par référence aux figures 10A à 10H. Au cours de l'actionnement, l'applicateur 602 est déplacé vers la position d'actionnement du dispositif de distribution 10. Lors de cette descente les premiers moyens de désengagement 606 parcourent la rampe des plots 609 avec une force suffisante pour faire sortir l'ergot 613 des moyens de verrouillage 603 de la seconde cavité 615 des moyens de blocage 605. En outre, le parcours de la rampe des plots 609 par les premiers moyens de désengagement 606 permet d'entraîner en translation horizontale les moyens de verrouillage 603 vers la position de verrouillage les moyens de verrouillage 603 (voir figures 10C et 10D). Ainsi, dans ce mode de réalisation, les premiers moyens de désengagement 606 forment également les moyens de retour des moyens de verrouillage 603 en position de verrouillage.

La descente de l'applicateur 602 est notamment permise grâce à la flexibilité des lames 612 qui se déforment élastiquement sous l'effet de la force transmise par les plots 609 lors du retour des moyens de verrouillage 603 en position de verrouillage (voir figure 10D).

L'applicateur 602 remonte ensuite et les lames 612 reprennent leurs conformations initiales. La situation de départ est donc retrouvée, c'est-à-dire que les moyens de verrouillage 603 sont en position de verrouillage et le dispositif de distribution 10 est en position de repos (voir figure 10E).

Dans le cas où l'utilisateur n'actionne pas le dispositif de distribution 10, soit parce que le déverrouillage a eu lieu accidentellement, soit parce qu'il ne désire finalement pas obtenir une dose de produit, il est alors nécessaire de pouvoir reverrouiller le dispositif de distribution 10, afin notamment d'éviter qu'une personne non autorisée l'utilise, sans pour autant actionner le dispositif de distribution 10, afin notamment de ne pas gaspiller du produit. Pour cela, on commande, par exemple grâce à un bouton et via les moyens de contrôle électroniques présents dans le socle 20, le parcours d'un courant électrique dans les seconds moyens de désengagement 607 formés par le fil rétractable (voir figure 10F). En se rétractant, ce fil rétractable 607, en se rétractant sous l'effet du courant électrique, entraîne en translation horizontale les moyens de verrouillage 603 vers la position de verrouillage (voir figure 10G). La force développée par le fil rétractable 607 est donc supérieure aux forces de frottement maintenant l'ergot 613 dans la seconde cavité 615 des moyens de blocage 605. Ainsi, les moyens de verrouillage 603 sont entraînés en translation horizontale jusqu'à la position de verrouillage sans qu'il y ait besoin d'actionner le dispositif de distribution 10. Comme indiqué précédemment on peut prévoir, alternativement ou cumulativement, que la commande du désengagement des moyens de verrouillage 603 soit effectuée autrement.

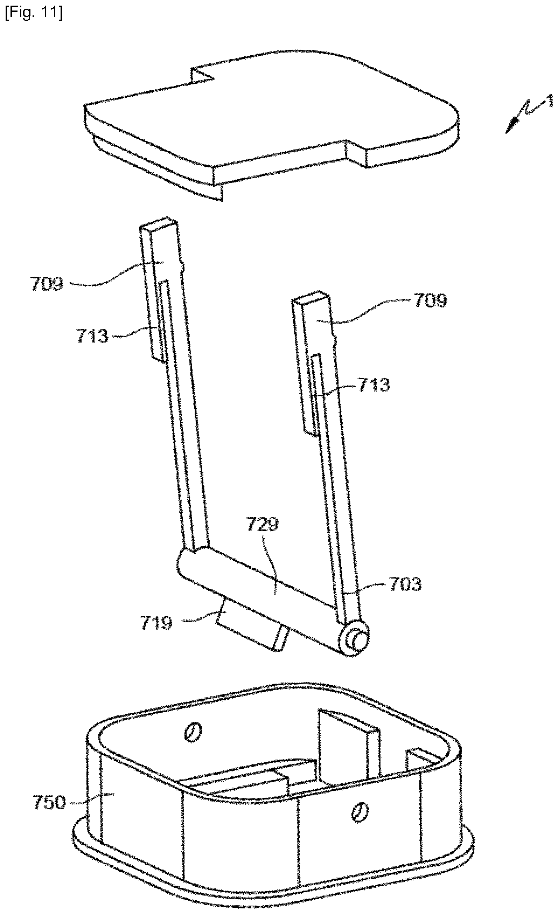

On a représenté sur les figures 11 à 12H un dispositif d'assistance 1 à l'utilisation d'un dispositif de distribution 10 de produit selon un septième mode de réalisation de l'invention.

Dans ce mode de réalisation, les moyens de verrouillage 703 ont une forme générale cylindrique circulaire à partir de laquelle des plots 709 s'étendent à partir de chacune des extrémités d'un cylindre 729 perpendiculairement à un axe longitudinal principal du cylindre 729 (voir figure 11). Les plots 709 ont une forme générale parallélépipédique. Dans le cas présent, les plots 709 sont au nombre de deux. On peut prévoir un nombre différent de plot 709, par exemple entre deux et vingt, de préférence entre deux et huit, voire entre deux et six. Une lame 713 s'étend depuis l'extrémité libre de chaque plot 709 en direction de l'autre extrémité du plot 709 (voir figure 11). Les moyens de verrouillage 703 comprennent en outre une protubérance 719 s'étendant radialement à partir d'une portion médiane du cylindre. Les moyens de verrouillage 703 sont montés sur le dispositif d'assistance 1 en rotation autour d'un axe perpendiculaire à l'axe longitudinal principal du dispositif d'assistance 1.

Le dispositif d'assistance 1 comprend un boîtier 750 apte à contenir une partie des moyens de verrouillage 703.

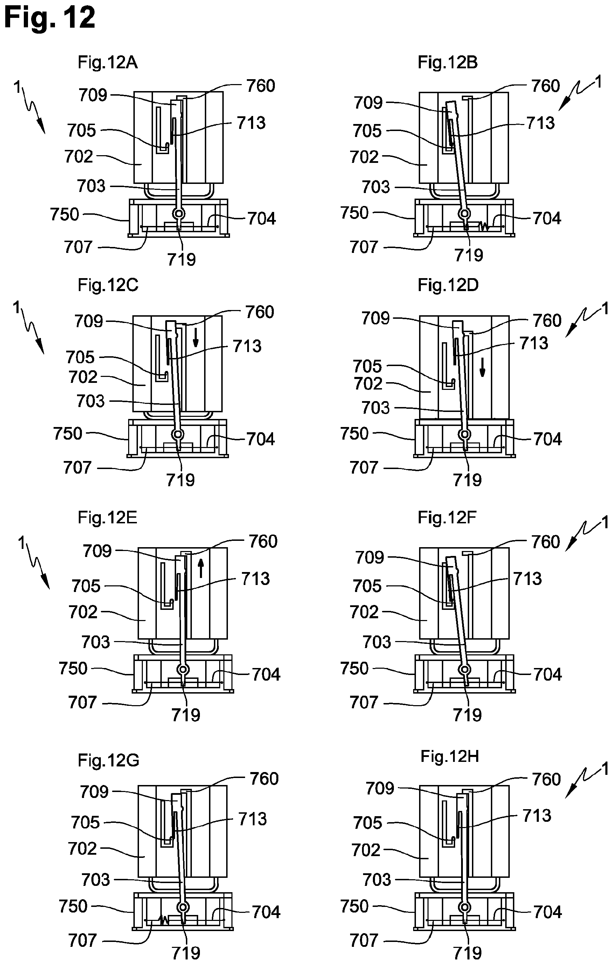

Les moyens de blocage 705 sont formés par deux reliefs s'étendant sur une paroi externe de l'applicateur 702 (voir figure 12A à 12H). Les deux reliefs 705 présentent chacun :
- une première portion rectiligne orientée parallèlement à un axe longitudinal principal de l'applicateur 702,
- une deuxième portion rectiligne qui fait directement suite à la première portion au niveau d'une extrémité proximale de celle-ci et qui est plus petite que celle-ci. La deuxième portion est orientée selon une direction perpendiculaire à l'axe longitudinal principal de l'applicateur 702, et
- une troisième portion rectiligne qui fait directement suite à une extrémité de la deuxième portion et qui est plus petite que celle-ci. La troisième portion est orientée selon une direction parallèle à l'axe longitudinal principal de l'applicateur 702 et s'étend depuis l'extrémité de la deuxième portion en direction distale.

En position de repos, les lames 713 des moyens de verrouillage 703 et les reliefs des moyens de blocage 705 ne sont pas en prises et sont à distance l'un de l'autre (voir figure 12A). En position de blocage, les lames 713 des moyens de verrouillage 703 sont en prise avec les reliefs des moyens de blocage 705 de manière à ce que les moyens de blocage 705 empêchent une rotation des moyens de verrouillage 703 en direction de la position de verrouillage (voir figure 12B).

Les moyens de retour (non représentés) configurés pour faire passer les moyens de verrouillage 703 de la position de déverrouillage à la position de verrouillage comprennent ici un ressort de torsion. Ce ressort de torsion est comprimé lors du passage des moyens de verrouillage 703 de la position de verrouillage à la position de déverrouillage et applique donc une force sur ceux-ci tendant à les ramener en position de verrouillage.

Dans le présent mode de réalisation, l'actionneur comprend un fil rétractable 704 dont une extrémité est fixée au boîtier 750 et une autre extrémité est fixée à la protubérance 719 des moyens de verrouillage 703. Ce fil rétractable 704 est apte, lorsqu'il est parcouru par un courant électrique, à se rétracter de manière à entraîner les moyens de verrouillage 703 en rotation et à les faire ainsi passer de la position de verrouillage (voir figure 12A) à la position de déverrouillage (voir figure 12B). On peut prévoir que l'actionneur 702 soit tout autre élément apte à remplir cette fonction.

L'applicateur 702 est configuré de manière à ce que lorsqu'il est actionné, les reliefs des moyens de blocage 705 s'éloignent des lames 713 des moyens de verrouillage 703 de manière à ce que ces deux éléments ne soient plus en prises et donc à ce que les moyens de verrouillage 703 ne soient plus bloqués en position de déverrouillage par les moyens de blocage 705 (voir figure 12C). Ainsi, l'applicateur 702 forme également les premiers moyens de désengagement. L'applicateur 702 comprend en outre des reliefs 760 sur les mêmes parois de l'applicateur 702 qui portent les reliefs des moyens de blocage 705. Ces reliefs 760 présentent chacun :
- une première portion rectiligne orientée parallèlement à un axe longitudinal principal de l'applicateur 702,
- une deuxième portion rectiligne qui fait directement suite à la première portion au niveau d'une extrémité distale de celle-ci et qui est plus petite que celle-ci. La deuxième portion est orientée selon une direction perpendiculaire à l'axe longitudinal principal de l'applicateur 702.

Les moyens de verrouillage 703 sont mobiles en rotation entre une position de verrouillage (voir figure 12A) dans laquelle les sommets des plots 709 sont chacun en regard et en appui contre un des reliefs 760 de l'applicateur 702 et une position de déverrouillage (voir figure 12B) dans laquelle les sommets des plots 709 ne sont pas en regard d'un des reliefs 760 de l'applicateur 702 et ni en appui contre ceux-ci.

Les seconds moyens de désengagement 707 comprennent ici un fil rétractable dont l'une des extrémités est fixée au boîtier 750, l'autre extrémité étant fixée à la protubérance 719 des moyens de verrouillage 703. Ainsi, le fil rétractable est apte, en se rétractant sous l'effet d'un courant électrique, à entraîner en rotation les moyens de verrouillage 703 de manière à ce que les lames 713 des moyens de verrouillage 703 ne soient plus en prise avec les reliefs des moyens de blocage 705.

Nous décrivons maintenant un mode de fonctionnement du dispositif d'assistance 1 selon ce septième mode de réalisation.

À l'état de repos, le dispositif de distribution 10 est dans sa position de repos et les moyens de verrouillage 703 sont en position de verrouillage (voir figure 10A). Comme indiqué précédemment, en position de verrouillage, les plots 709 des moyens de verrouillage 703 sont en regard et en appui contre les reliefs 760 de l'applicateur 702. Ces reliefs 760 de l'applicateur 702 sont en butée contre les sommets des plots 709 lorsque l'utilisateur tente d'utiliser le dispositif de distribution 10 sans l'avoir déverrouillé ce qui empêche l'actionnement du dispositif de distribution 10. Lorsque l'utilisateur souhaite utiliser le dispositif de distribution 10, il place son doigt sur le lecteur d'empreinte 22 afin de le déverrouiller de la même manière que pour les précédents modes de réalisation. Lorsque l'empreinte digitale de l'utilisateur est authentifiée, les moyens de contrôle électroniques via les composants électroniques présents dans le socle 20 font parcourir un courant électrique dans le fil rétractable 704 de manière à ce que celui-ci se rétracte. Cette rétractation entraîne la rotation des moyens de verrouillage 703 de la position de verrouillage (voir figure 12A) vers la position de déverrouillage (voir figure 12B). Les lames 713 présentent une élasticité suffisante pour que, lorsqu'elles viennent en butée contre une partie des reliefs des moyens de blocage 705 sous l'effet du fil rétractable 704, elles se déforment et passent de l'autre côté du relief (voir figures 12A et 12B). Les lames 713 sont en outre assez rigides pour rester bloquées dans cette position. Les moyens de verrouillage 703 sont alors bloqués en position de déverrouillage.

En position de déverrouillage, les reliefs 760 de l'applicateur 702 ne sont plus en regard des sommets des plots 709 des moyens de verrouillage 703. Ainsi, ces reliefs 760 ne butent plus contre les sommets des plots 709 lorsqu'un utilisateur tente d'actionner le dispositif de distribution 10. L'actionnement du dispositif de distribution 10 est donc possible.

Deux options sont alors envisageables : soit l'utilisateur actionne le dispositif de distribution 10, soit il ne l'actionne pas.

Dans le cas où l'utilisateur actionne le dispositif de distribution 10, il déplace l'actionneur 702 en direction proximale, c'est-à-dire vers le bas par référence aux figures 11 à 12H. Au cours de l'actionnement, l'applicateur 702 est déplacé vers la position d'actionnement du dispositif de distribution 10. Au bout d'une course prédéterminée, les reliefs des moyens de blocage 705 se sont suffisamment éloignés des lames 713 pour ne plus être en prise avec celles-ci. Les moyens de retour formés par le ressort de torsion entraînent alors les moyens de verrouillage 703 en rotation en direction de la position de verrouillage. Toutefois, du fait de la descente de l'applicateur 702 les moyens de verrouillage 703 ne peuvent pas retrouver tout de suite la position de verrouillage et les plots 709 viennent en butée contre les reliefs 760 (voir figure 12D). Lorsque l'applicateur a fini sa remontée, les moyens de retour entraînent à nouveau les moyens de verrouillage 703 en rotation, cette fois jusqu'en position de verrouillage. La situation de départ est donc retrouvée, c'est-à-dire que les moyens de verrouillage 703 sont en position de verrouillage et le dispositif de distribution 10 est en position de repos.

Dans le cas où l'utilisateur n'actionne pas le dispositif de distribution 10, soit parce que le déverrouillage a eu lieu accidentellement, soit parce qu'il ne désire finalement pas obtenir une dose de produit, il est alors nécessaire de pouvoir reverrouiller le dispositif de distribution 10, afin notamment d'éviter qu'une personne non autorisée l'utilise, sans pour autant actionner le dispositif de distribution 10, afin notamment de ne pas gaspiller du produit. Pour cela, on commande, par exemple grâce à un bouton et via les moyens de contrôle électroniques présents dans le socle 20, le parcours d'un courant électrique dans les seconds moyens de désengagement 707 formés par le fil rétractable (voir figure 12F). En se rétractant, ce fil rétractable 707 déforme entraîne les moyens de verrouillage 703 en rotation en direction de la position de verrouillage (voir figure 12G). Les moyens de verrouillage 703 retournent ainsi en position de verrouillage (voir figure 12H) sans qu'il y ait besoin d'actionner le dispositif de distribution 10. Comme indiqué précédemment on peut prévoir, alternativement ou cumulativement, que la commande du désengagement des moyens de verrouillage 703 soit effectuée autrement.

L'invention n'est pas limitée aux modes de réalisation présentés et d'autres modes de réalisation apparaîtront clairement à l'homme du métier.

## Revendications

1. Dispositif d'assistance (1) à l'utilisation d'un dispositif de distribution (10) d'un produit, la distribution du produit étant mise en oeuvre par déplacement ou déformation d'une partie mobile (102, 202, 302, 402, 502, 602, 702) du dispositif de distribution (10) entre une position de repos et une position d'activation, le dispositif d'assistance (1) comprenant :
- des moyens de solidarisation au dispositif de distribution (10) de produit,
- des moyens de verrouillage (103, 203, 303, 403, 503, 603, 703) aptes à empêcher la distribution du produit,
- un actionneur (104, 204, 304, 404, 504, 604, 704) configuré pour faire passer les moyens de verrouillage (103, 203, 303, 403, 503, 603, 703) d'une position de verrouillage dans laquelle ils empêchent la distribution du produit à une position de déverrouillage dans laquelle ils n'empêchent pas la distribution du produit,
- des moyens de retour (240, 340, 440) configurés pour faire passer les moyens de verrouillage (103, 203, 303, 403, 503, 603, 703) de la position de déverrouillage à la position de verrouillage, en dehors de la position d'activation du dispositif de distribution (10) du produit,
- des moyens de blocage (105, 205, 305, 405, 505, 605, 705) configurés pour bloquer les moyens de verrouillage (103, 203, 303, 403, 503, 603, 703) en position de déverrouillage,
- des premiers moyens de désengagement (106, 206, 306, 406, 506, 606) des moyens de blocage (105, 205, 305, 405, 505, 605, 705) configurés pour être déclenchés lors de l'activation du dispositif de distribution (10) de produit,
**caractérisé en ce qu'**il comprend en outre :
- des seconds moyens de désengagement (107, 207, 307, 407, 507, 607, 707) des moyens de blocage (105, 205, 305, 405, 505, 605, 705) configurés pour être déclenchés indépendamment de l'activation du dispositif de distribution (10) de produit.

2. Dispositif d'assistance (1) selon la revendication 1, dans lequel l'actionneur (104, 704) comprend au moins un fil rétractable.

3. Dispositif d'assistance (1) selon la revendication 1, dans lequel l'actionneur comprend un moteur.

4. Dispositif d'assistance (1) selon l'une quelconque des revendications précédentes, dans lequel les seconds moyens de désengagement (107, 207, 307, 407, 507, 607, 707) comprennent au moins un fil rétractable.

5. Dispositif d'assistance (1) selon l'une quelconque des revendications précédentes, dans lequel les seconds moyens de désengagement comprennent un moteur.

6. Dispositif d'assistance (1) selon l'une quelconque des revendications précédentes, dans lequel les seconds moyens de désengagement (107, 207, 307, 407, 507, 607, 707) sont configurés pour être activés après qu'un temps prédéterminé se soit écoulé à partir d'un passage des moyens de verrouillage (103, 203, 303, 403, 503, 603, 703) en position de déverrouillage.

7. Dispositif d'assistance (1) selon l'une quelconque des revendications précédentes, comprenant en outre au moins un capteur d'inclinaison du dispositif de distribution (10), dans lequel les seconds moyens de désengagement (107, 207, 307, 407, 507, 607, 707) sont configurés pour être activés lorsque, les moyens de verrouillage (103, 203, 303, 403, 503, 603, 703) étant en position de déverrouillage, une mesure d'inclinaison du dispositif de distribution (10) mesurée par le capteur d'inclinaison atteint un seuil prédéterminé pendant un laps de temps prédéterminé.

8. Dispositif d'assistance (1) selon l'une quelconque des revendications précédentes, dans lequel les moyens de verrouillage (103, 403, 503) sont configurés pour passer de la position de déverrouillage à la position de verrouillage en suivant un mouvement de rotation.

9. Dispositif d'assistance (1) selon l'une quelconque des revendications précédentes, dans lequel le dispositif d'assistance (1) comprend au moins un cran apte à empêcher le retour de la partie mobile (102, 202, 302, 402, 502, 602, 702) du dispositif de distribution (10) vers la position de repos après un déplacement selon une course prédéterminée de la partie mobile (102, 202, 302, 402, 502, 602, 702) du dispositif de distribution (10), la course prédéterminée étant plus courte qu'une course totale correspondant à la course aboutissant à la position d'activation du dispositif de distribution (10).

## Patentansprüche

1. Vorrichtung (1) zur Unterstützung der Verwendung einer Abgabevorrichtung (10) für ein Produkt, wobei die Abgabe des Produkts durch Verschieben oder Verformen eines beweglichen Teils (102, 202, 302, 402, 502, 602, 702) der Abgabevorrichtung (10) zwischen einer Ruheposition und einer Aktivierungsposition erfolgt, wobei die Unterstützungsvorrichtung (1) aufweist:
- Mittel zur festen Verbindung mit der Produkt-Abgabevorrichtung (10),
- Verriegelungsmittel (103, 203, 303, 403, 503, 603, 703), die eingerichtet sind, die Abgabe des Produkts zu verhindern,
- einen Aktuator (104, 204, 304, 404, 504, 604, 704), der konfiguriert ist, die Verriegelungsmittel (103, 203, 303, 403, 503, 603, 703) von einer Verriegelungsposition, in der sie die Ausgabe des Produkts verhindern, in eine Entriegelungsposition, in der sie die Ausgabe des Produkts nicht verhindern, zu bewegen,
- Rückstellmittel (240, 340, 440), die konfiguriert sind, die Verriegelungsmittel (103, 203, 303, 403, 503, 603, 703) aus der Entriegelungsstellung in die Verriegelungsstellung zu bringen, außerhalb der Aktivierungsstellung der Produkt-Abgabevorrichtung (10),
- Blockiermittel (105, 205, 305, 405, 505, 605, 705), die konfiguriert sind, die Verriegelungsmittel (103, 203, 303, 403, 503, 603, 703) in der Entriegelungsposition zu blockieren,
- erste Mittel zum Lösen (106, 206, 306, 406, 506, 606) der Blockiermittel (105, 205, 305, 405, 505, 605, 705), die konfiguriert sind, bei der Aktivierung der Produkt-Abgabevorrichtung (10) ausgelöst zu werden,
**dadurch gekennzeichnet, dass** sie außerdem aufweist:
- zweite Mittel zum Lösen (107, 207, 307, 407, 507, 607, 707) der Blockiermittel (105, 205, 305, 405, 505, 605, 705), die konfiguriert sind, unabhängig von der Aktivierung der Produkt-Abgabevorrichtung (10) ausgelöst zu werden.

2. Unterstützungsvorrichtung (1) nach Anspruch 1, wobei der Aktuator (104, 704) mindestens einen einziehbaren Draht aufweist.

3. Unterstützungsvorrichtung (1) nach Anspruch 1, wobei der Aktuator einen Motor aufweist.

4. Unterstützungsvorrichtung (1) nach einem der vorhergehenden Ansprüche, wobei die zweiten Lösemittel (107, 207, 307, 407, 507, 607, 707) mindestens einen einziehbaren Draht aufweisen.

5. Unterstützungsvorrichtung (1) nach einem der vorhergehenden Ansprüche, wobei die zweiten Lösemittel einen Motor aufweisen.

6. Unterstützungsvorrichtung (1) nach einem der vorhergehenden Ansprüche, wobei die zweiten Lösemittel (107, 207, 307, 407, 507, 607, 707) konfiguriert sind, aktiviert zu werden, nachdem eine vorbestimmte Zeit ab einem Übergang der Verriegelungsmittel (103, 203, 303, 403, 503, 603, 703) in die Entriegelungsposition verstrichen ist.

7. Unterstützungsvorrichtung (1) nach einem der vorhergehenden Ansprüche, die außerdem mindestens einen Sensor für die Neigung der Abgabevorrichtung (10) aufweist, wobei die zweiten Lösemittel (107, 207, 307, 407, 507, 607, 707) konfiguriert sind, aktiviert zu werden wenn, die Verriegelungsmittel (103, 203, 303, 403, 503, 603, 703) in der Entriegelungsposition sind, ein Neigungsmaß der Abgabevorrichtung (10), das von dem Neigungssensor gemessen wird, während einer vorbestimmten Zeitdauer einen vorbestimmten Schwellenwert erreicht.

8. Unterstützungsvorrichtung (1) nach einem der vorhergehenden Ansprüche, wobei die Verriegelungsmittel (103, 403, 503) konfiguriert sind, sich durch eine Drehbewegung von der Entriegelungsposition in die Verriegelungsposition zu bewegen.

9. Unterstützungsvorrichtung (1) nach einem der vorhergehenden Ansprüche, wobei die Unterstützungsvorrichtung (1) mindestens eine Raste aufweist, die eingerichtet ist, die Rückkehr des beweglichen Teils (102, 202, 302, 402, 502, 602, 702) der Abgabevorrichtung (10) in die Ruheposition zu verhindern, nachdem der bewegliche Teil (102, 202, 302, 402, 502, 602, 702) der Abgabevorrichtung (10) um einen vorbestimmten Hub verschoben, wobei der vorbestimmte Hub kürzer ist als ein Gesamthub, der dem Hub entspricht, der zu der Aktivierungsposition der Abgabevorrichtung (10) führt.

## Claims

1. Assistance device (1) for assisting with the use of a product dispensing device (10) for dispensing a product, the product dispensing being achieved by displacement or deformation of a moving part (102, 202, 302, 402, 502, 602, 702) of the dispensing device (10) between a rest position and an activation position, the assistance device (1) comprising:
- engaging means to the product dispensing device (10),
- locking means (103, 203, 303, 403, 503, 603, 703) capable of preventing the dispensing of the product,
- an actuator (104, 204, 304, 404, 504, 604, 704) configured to switch the locking means (103, 203, 303, 403, 503, 603, 703) from a locked position in which they prevent the dispensing of the product to an unlocked position in which they do not prevent the dispensing of the product,
- return means (240, 340, 440) configured to switch the locking means (103, 203, 303, 403, 503, 603, 703) from the unlocked position to the locked position, out of the activation position of the product dispensing device (10),
- blocking means (105, 205, 305, 405, 505, 605, 705) configured to block the locking means (103, 203, 303, 403, 503, 603, 703) in the unlocked position,
- first disengagement means (106, 206, 306, 406, 506, 606) for disengaging the blocking means (105, 205, 305, 405, 505, 605, 705) configured to be triggered upon the activation of the product dispensing device (10),
**characterised in that** it further comprises:
- second disengagement means (107, 207, 307, 407, 507, 607, 707) for disengaging the blocking means (105, 205, 305, 405, 505, 605, 705) configured to be triggered independently of the activation of the product dispensing device (10).

2. Assistance device (1) according to claim 1, wherein the actuator (104, 704) comprises at least one muscle wire.

3. Assistance device (1) according to claim 1, wherein the actuator comprises a motor.

4. Assistance device (1) according to any one of the preceding claims, wherein the second disengagement means (107, 207, 307, 407, 507, 607, 707) comprise at least one muscle wire.

5. Assistance device (1) according to any one of the preceding claims, wherein the second disengagement means comprise a motor.

6. Assistance device (1) according to any one of the preceding claims, wherein the second disengagement means (107, 207, 307, 407, 507, 607, 707) are configured to be activated after a predetermined time has elapsed from the switching of the locking means (103, 203, 303, 403, 503, 603, 703) to the unlocked position.

7. Assistance device (1) according to any one of the preceding claims, further comprising at least one inclination sensor for measuring the inclination of the dispensing device (10), wherein the second disengagement means (107, 207, 307, 407, 507, 607, 707) are configured to be activated when, with the locking means (103, 203, 303, 403, 503, 603, 703) in the unlocked position, a measurement of the inclination of the dispensing device (10) measured by the inclination sensor reaches a predetermined threshold during a predetermined period of time.

8. Assistance device (1) according to any one of the preceding claims, wherein the locking means (103, 403, 503) are configured to switch from the unlocked position to the locked position by performing a rotational movement.

9. Assistance device (1) according to any one of the preceding claims, wherein the assistance device (1) comprises at least one indentation capable of preventing the moving part (102, 202, 302, 402, 502, 602, 702) of the dispensing device (10) from returning to the rest position after a displacement along a predetermined stroke of the moving part (102, 202, 302, 402, 502, 602, 702) of the dispensing device (10), the predetermined stroke being shorter than a total stroke corresponding to the stroke leading to the activation position of the dispensing device (10).
